# EUROPEAN PATENT SPECIFICATION

(11) **EP 0 751 957 B1**
(45) Date of publication and mention of the grant of the patent: **25.02.2004**
(21) Application number: 95912506.3
(22) Date of filing: 21.03.1995
(51) Int. Cl.: C07K 14/35, C12N 9/02, C12N 15/31, C12N 15/53, C12N 15/63, C12N 5/08, C07K 16/18, A61K 38/08, G01N 33/68

(54) **PEPTIDE FRAGMENTS OF MICROBIAL STRESS PROTEINS AND PHARMACEUTICAL COMPOSITION MADE THEREOF FOR THE TREATMENT AND PREVENTION OF INFLAMMATORY DISEASES**
PEPTIDFRAGMENTE VON MIKROBIELLEN STRESSPROTEINEN UND DARAUS HERGESTELLTE PHARMAZEUTISCHE ZUSAMMENSETZUNGEN FÜR DIE BEHANDLUNG UND VORBEUGUNG VON ENTZÜNDUNGSERKRANKUNGEN
FRAGMENTS PEPTIDIQUES DE PROTEINES DE STRESS MICROBIENNES, ET COMPOSITION PHARMACEUTIQUE A BASE DE CEUX-CI DESTINEE AU TRAITEMENT ET A LA PREVENTION DES MALADIES INFLAMMATOIRES

(30) Priority: 21.03.1994 EP 94200721; 22.03.1994 EP 94200738; 10.10.1994 EP 94202927
(43) Date of publication of application: 08.01.1997
(73) Proprietor: Rijksuniversiteit Utrecht, 3584 CS Utrecht (NL)
(72) Inventor: ANDERTON, Stephen, Mark, Bristol B5 81 TD (GB); VAN DER ZEE, Ruurd, NL-3572 CA Utrecht (NL); VAN EDEN, Willem, NL-3723 HB Bilthoven (NL)
(74) Representative: van Westenbrugge, Andries
(86) International application number: PCT/NL1995/000108
(87) International publication number: WO 1995/025744

(56) References cited:
- EP-A- 0 262 710
- EP-A- 0 322 990
- WO-A-88/06591
- WO-A-90/10449
- WO-A-92/04049
- EMBO JOURNAL, vol. 6, no. 5, EYNSHAM, OXFORD GB, pages 1245-1249, J.R. LAMB ET AL. 'Mapping of T cell epitopes using recombinant antigens and synthetic peptides'
- JOURNAL OF IMMUNOLOGY, vol. 141, no. 8, 15 October 1988 BALTIMORE US, pages 2749-2754, F. OFTUNG ET AL. 'Epitopes of the Mycobacterium Tuberculosis 65-Kilodalton Protein Antigen as Recognized by Human T-Cells'
- PEPT. CHEM. (1986), VOLUME DATE 1985, 23RD, 357-62 CODEN: PECHDP;ISSN: 0388-3698, KITAMURA, KAZUO ET AL 'Calmodulin binding peptides identified in porcine brain'
- ANDERTON: 'Differential Mycobacterial 65-kDa Heat Shock Protein T Cell Epitope Recognition...' JOURNAL OF IMMUNOLOGY vol. 152, no. 7, 01 April 1994, pages 3656 - 3664
- MUNK ET AL: 'T Lymphocytes from healthy individuals with specificity to self-epitopes shared by the mycobacterial and human 65-kilodalton heat shock protein.' JOURNAL OF IMMUNOLOGY vol. 143, no. 9, 01 November 1989, pages 2844 - 2849
- QUAYLE ET AL: 'Peptide recognition, Tcell receptor usage and HLA restriction elements of human heat-shock protein (hsp) 60 and mycobacterial 65-kDa hsp-reactive T cell clones from rheumatoid synovial flow' EUROPEAN JOURNAL OF IMMUNOLOGY vol. 22, 1992, pages 1315 - 1322

## Description

### Field of the invention

The invention pertains to the use of peptides containing a part of the aminoacid sequence of a stress protein having conserved homologues in microorganisms and mammals, which peptides are capable of immunising against arthritis and other inflammatory diseases and/or curing such diseases, as well as to the use of nucleotide sequences encoding such peptides.

### Background

Stress proteins have been described as useful for providing immunity against non-viral infections or for inducing immune tolerance. WO 89/12455 discloses a vaccine containing a mycobacterial stress protein for immune prophylaxis against autoimmune diseases, especially rheumatoid arthritis.

Adjuvant arthritis (AA) is an extensively studied model of human rheumatoid arthritis (RA) or reactive arthritis. As the pathogenic mechanisms underlying RA are still unclear, extensive use is made of experimental rodent arthritis models. Lewis rats are susceptible to arthritis following administration of various arthritogenic preparations including heat-killed M. tuberculosis (Mt) suspended in IFA (adjuvant arthritis or, AA) (1), streptococcal cell walls (SCW-arthritis), collagen type II and the lipoidal amine CP20961. The cellular basis for AA induction was demonstrated by the passive transfer of the disease to naive rats using splenocytes from arthritic rats (2). Induction of disease in irradiated naive rats by administration of the Mt-reactive T cell clone A2b was reported (3,4). The Ag-specificity of A2b was identified as residues 180-188 of the mycobacterial 65 kDa heat shock protein (hsp65) (5).

EP-A-262710 describes vaccines against autoimmune diseases containing a peptide corresponding to the aminoacid sequence 171-240 or parts thereof of mycobacterial hsp65. EP-A-322990 describes similar vaccines based on the sequence 180-188 of hsp65. Mutant peptides corresponding to aminoacid sequence 180-186 of hsp65 and T cells reactive to such mutant peptides are disclosed in WO-A-9204049. According to WO-A-9403208 synthetic peptides derived from human hsp65 sequences 458-474 and 437-448 and the mycobacterial analogue 430-446 increase the immunogenicity of poorly immunogenic antigens.

WO 88/06591 discloses mycobacterial peptides for use in vaccines against mycobacterial infections, including the sequence 231-245 of hsp65 of *M. tuberculosis.* The mapping of T cell epitopes using mycobacterial antigens, including sequence 112-132 of hsp65 is decribed in EMBO J. 6, 1245-1249 (1987). The role of the sequences 211-225, 231-245 and others of mycobacterial hsp65 in human T cell recognition is reported in J. Immunol. 141, 2749-2754 (1988). WO 90/10449 teaches the use of the sequence of the human hsp65 corresponding to the mycobacterial sequence 410-432 in the treatment of insulin dependent diabetes mellitus.

Munk et al (14) have concluded that the demonstration of T cells with specificity to self-epitopes of hsp65 in vitro is not indicative for autoimmune diseases, but that, if at certain stages of infection such T cells are activated by crossreactive microbial epitopes, these could cause autoimmune responses. Quayle et al (17) describe pentadecapeptides of hsp 65, including 241-255 and 251-265, and their recognition by T cells, and they suggest that autoimmune disease could possibly be triggered by bacterial epitopes with homology to self protein, but that there are alternative interpretations of their data.

Attempts to induce AA by immunisation with hsp65 alone proved unsuccessful. Instead, this approach conferred resistance to subsequently attempted induction of AA with whole Mt (5,6). This protective effect is believed to be mediated by T cells specific for hsp65 (7). Preimmunisation with mycobacterial hsp65 has subsequently been reported to confer protection against other forms of experimental arthritis induced with streptococcal cell walls (8), collagen type II (6,9), or synthetic adjuvants such as CP20961 (6) and pristane (10).

Mycobacterial hsp65 belongs to the hsp60 family of heat shock proteins which is highly conserved throughout evolution, and shares 48% aminoacid identity with the mammalian homologue, P1 or hsp60 (11). Expression of mammalian hsp60 is known to be upregulated as a physiological response to various stressful stimuli, and has been shown to be elevated in inflamed synovia of patients with RA (12), or juvenile chronic arthritis (JCA, ref.13).

### Description of the invention

The invention is based on the finding that protective epitopes for prevention and treatment of inflammatory diseases are located at relatively short regions (about 5 to 15 aminoacids) of stress proteins, which regions are highly conserved between microorganisms and mammals. In addition to the high degree of identity in the protective epitopes, the proteins are, more generally, highly conserved between microorganisms and mammals.

Anderton et al. (the present inventors), *J*. *Immunol.* 1994 (April), 152: 3656, reported on the T cell events involved in protective effects of hsp65 preimmunisation.

The term "stress protein" is used here to denote enzymes or proteins that exhibit a raised level of synthesis during inflammation or other stress stimuli in cells residing at the site of such inflammation or stress condition. Normally, stress proteins are constitutively expressed, e.g. to exert house-keeping and metabolic functions in cells. In this description, a "microbial stress protein" is to be understood as a microbial homologue of a mammalian stress protein. Inflammation may result from infection, autoimmune disease, tumour growth, transplant rejection or tissue trauma. Other stress stimuli include increased temperature (up to 45□C), drugs, heavy metals, exogenous organic substances, oxidants, bacterial toxins, LPS, stress inducing lipoproteins, mitogens (PHA, ConA) and cytokines, such as IL1, IL2, TNFα, INFα and β, IL6 and IL12.

Raised synthesis can lead to a raised level of excretion or release of such proteins by cells or a raised level of presentation to the immune system. Raised synthesis can be detected e.g. by Northern blotting to measure raised levels of mRNA, or by measuring increased amounts of protein by using standard assays to quantify cell proteins or by using Western blotting with protein-specific antibodies.

Conservation of stress proteins is defined as exhibiting an overall aminoacid sequence identity between the microbial and the mammalian proteins at least 25%, preferably at least 40% of the aminoacids. In addition, areas of at least 75, up to e.g. 100, consecutive aminoacids must have a sequence identity between the microbial and the mammalian homologues of at least 40%, and more preferably at least 50% of the aminoacids. A further criterion that may applied is that the microbial and the mammalian homologues exhibit at least five areas of at least 10, up to about 15, consecutive aminoacids with a sequence identity of at least 40%, preferably at least 50%, and more preferably at least 60% of the aminoacids.

The protective peptides are derived from sequences comprising at least aminoacids which are in the same relative position as the same aminoacids in a T cell epitope of the microbial heat-shock protein, which epitope contains at least 4 consecutive aminoacids which are identical with the corresponding mammalian stress protein aminoacids.

The peptide to be used according to the invention thus contains a T cell epitope of the heat-shock protein and comprises up to 30 aminoacids. In addition, the peptide may comprise other sequences, whether or not derived from the microbial stress protein. Preferably, the peptide does not comprise the entire aminoacid sequence of the stress protein, but lacks at least one of the epitopes with insufficient homology. Thus, the peptide preferably does not contain a section of at least 5 aminoacids, especially at least 8 aminoacids, corresponding to a T cell epitope of said stress protein, when this epitope contains less than 3, especially less than 4, consecutive aminoacids which are identical with the corresponding mammalian stress protein aminoacids. Also, two or more of such low-homology epitopes may preferably be absent in the peptide used according to the invention.

Microorganisms and microbes include bacteria, but also protozoal and eukaryotic parasites. Mammals include humans, mouse and rat.

Stress proteins include non-enzyme proteins including heat-shock proteins (hsp) such as mycobacterial hsp65, hsp 60 (GroEL), DnaJ, hsp70 family (DnaK), ubiquitin, hsp10 (GroES), low molecular weight HSP's (20-30 kDa), hsp47, hsp56, TCP-1 (T complex peptide), hsp90, hsp104/110.

As further examples of non-enzyme stress proteins, histone H3 (J. Immun. 142: 1512, 1989), histone H2A, kinesin-related protein, acidic ribosomal proteins, crystallin, calreticulin (Michalak et al., Biochem. J. 285: 681-692) and the 18 kDa histon-like protein of Chlamidia may be mentioned.

The 18 kDa Chlamidia histon-like protein, a stress-inducible microbial antigen, was shown to be recognised by T cells in patients with reactive arthritis. In experimental models of autoimmunity, exposure to bacterial antigens has been found to lead to protective immune responses. Bordetella pertussis or Mycobacterium tuberculosis may induce protection against experimental autoimmune encephalomyelitis (EAE) (Lehman, Ben-Nun, J. Autoimm. 5: 675, 1992). M. bovis may protect against diabetes in the rat (M.W.J. Sadelain et al., J. Autoimmun. 3: 671-680; 1990).

Thus, the invention relates to the use of peptides providing protection against inflammatory diseases, which peptides correspond to at least a part of a T cell epitope for a microbial protein having a conserved mammalian stress protein counterpart, as further defined in the appending claims.

Peptides based on sequences of the mammalian protein itself are not suitable, since in the case of immunogenicity of such epitopes these will constitute cryptic epitopes which do not elicit protective responses recognising the whole mammalian self-protein. The reason for this is believed to be the fact that responses to epitopes of naturally processed self-proteins would be subject to thymic negative selection or peripheral tolerance. Protective effects in inflammation will be practically restricted to peptides representing microbial (homologous) epitopes that induce relatively low-affinity cross-reactive responses to mammalian self-proteins. For such low-affinity responses, negative selection or peripheral tolerance may be absent.

The invention also provides a method of producing a peptide as defined above, comprising the steps of:
a) selecting a microbial heat-shock protein having a conserved mammalian stress protein homologue, wherein the overall aminoacid sequence identity between the microbial and the mammalian homologues is at least 25%, preferably at least 40%, the sequence identity between the microbial and the mammalian homologues of an area of at least 75 consecutive aminoacids is at least 40%, preferably at least 50%, and the sequence identity between the microbial and the mammalian homologues of at least five areas of at least 10 consecutive aminoacids is at least 40%, preferably at least 50 or even 60%;
b) preparing peptides of up to 30 aminoacids comprising at least 5 aminoacids which are in the same relative position as the same aminoacids of said stress protein, of which a series of at least 4 consecutive aminoacids is identical both to a series of aminoacids of the selected microbial protein and to the corresponding series of mammalian stress protein aminoacids;
c) screening the prepared peptides for the presence of a T cell epitope, as evidenced by a T cell response by one or more individuals, not necessarily by all individuals of a population under investigation.

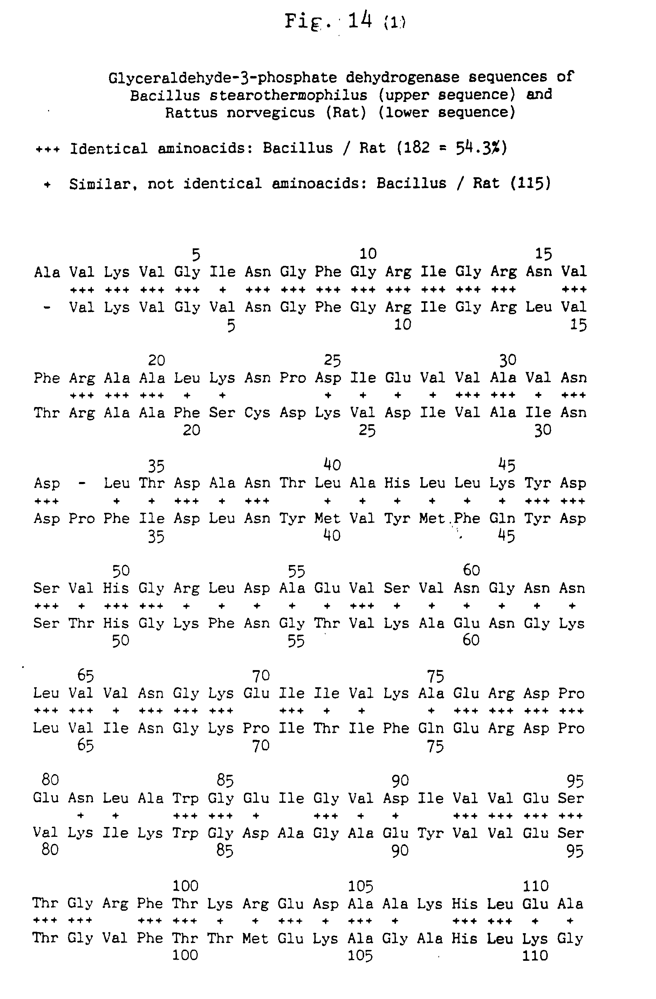

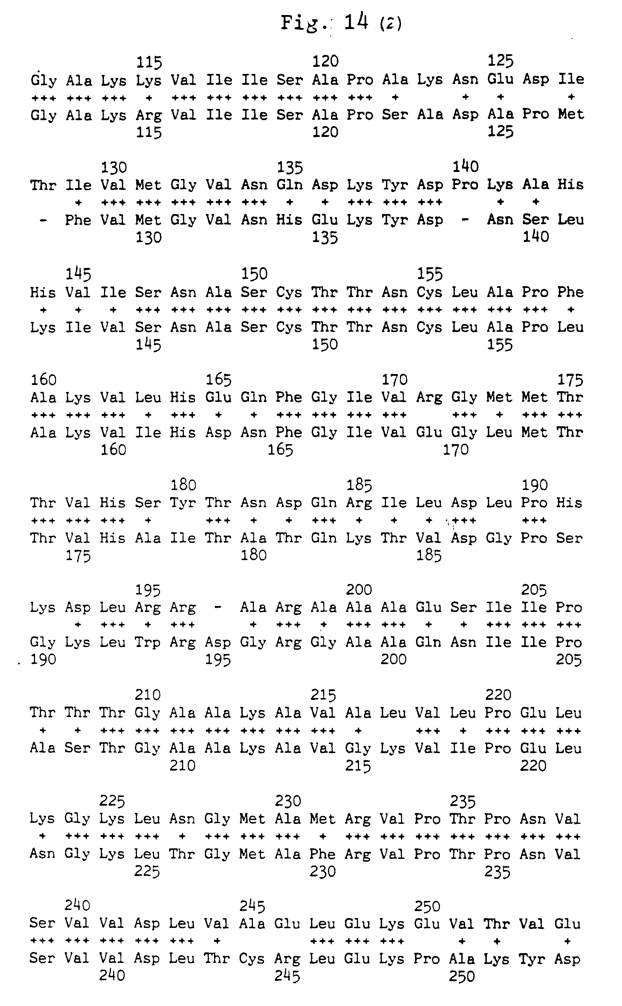

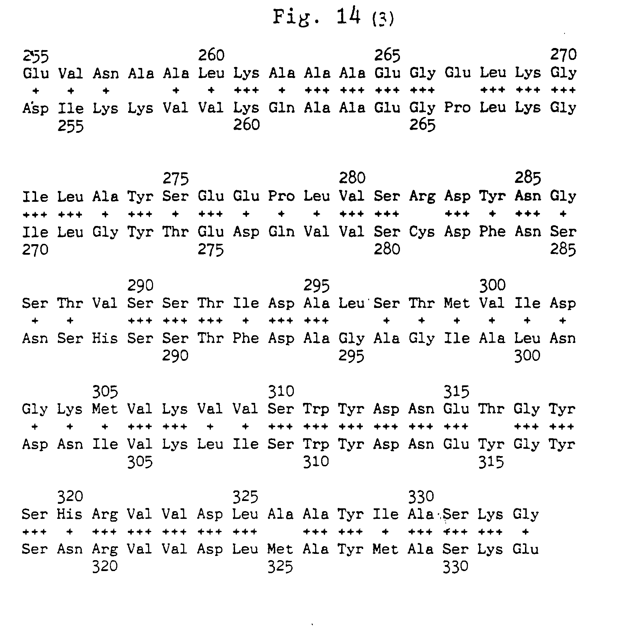

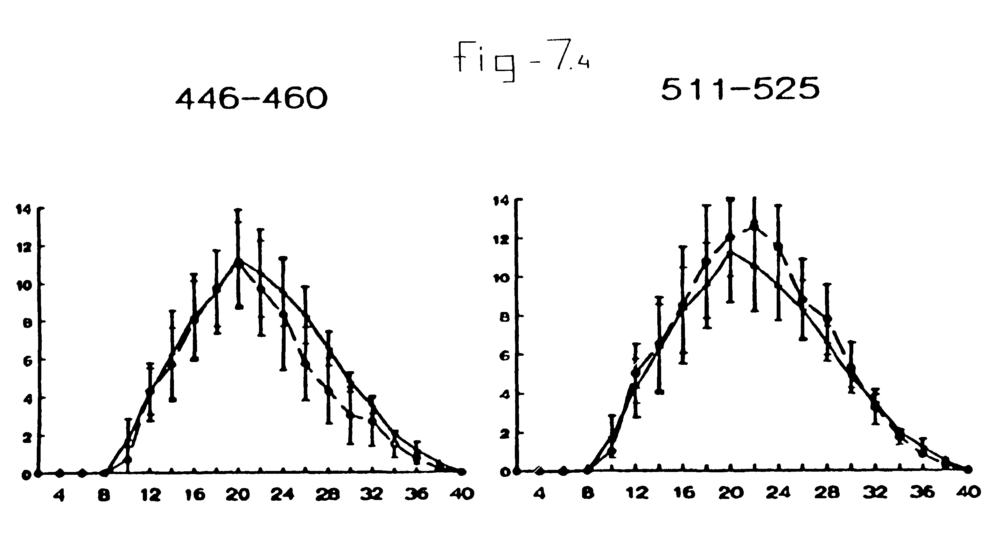

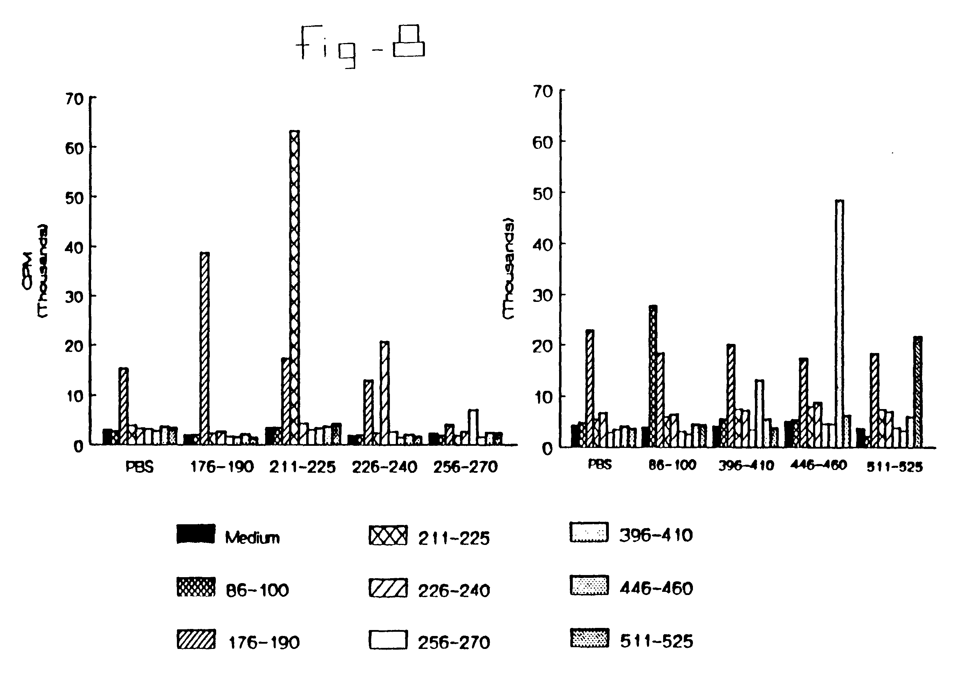

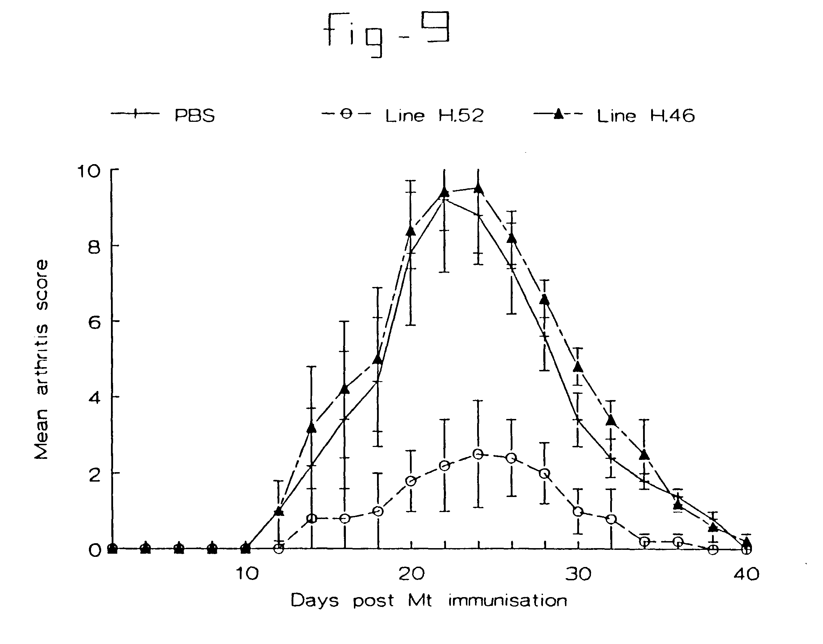

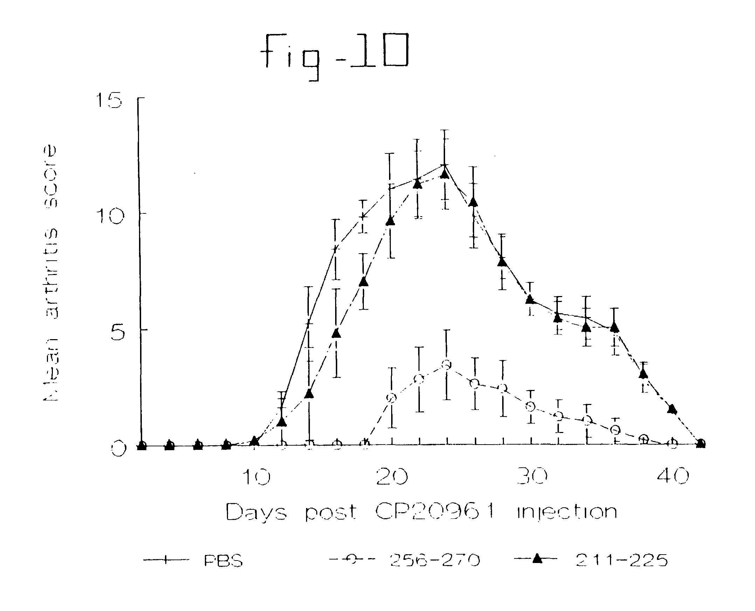

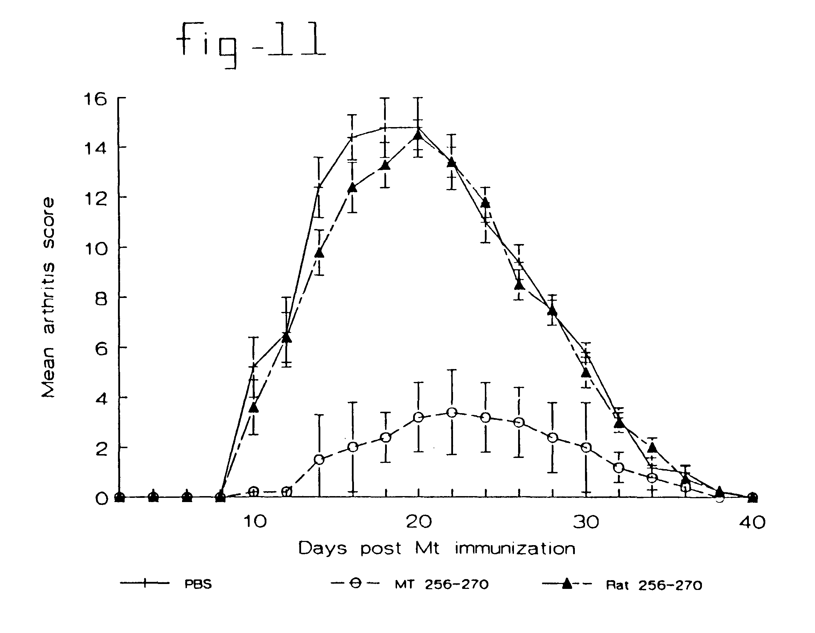

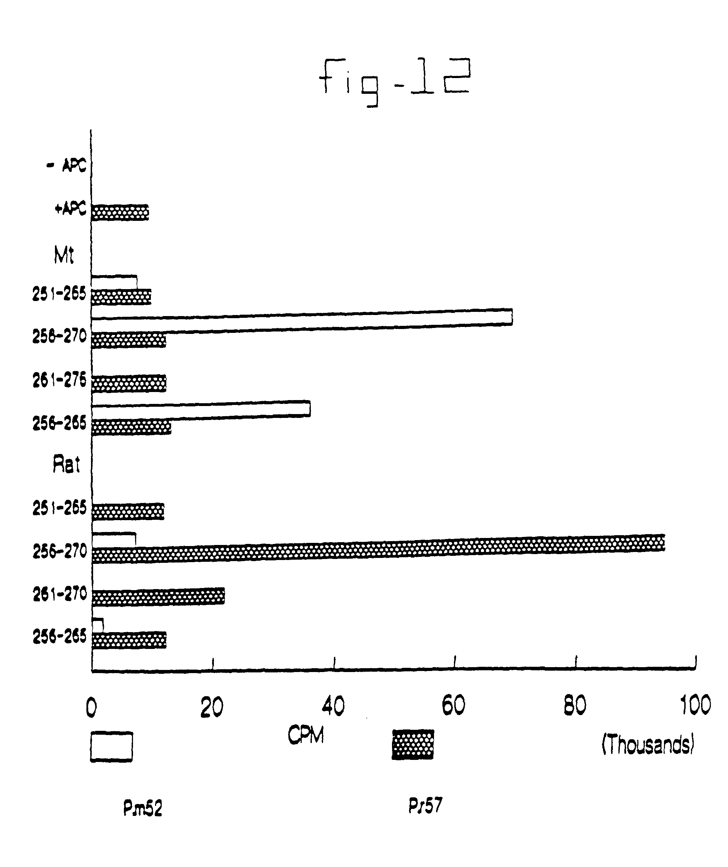

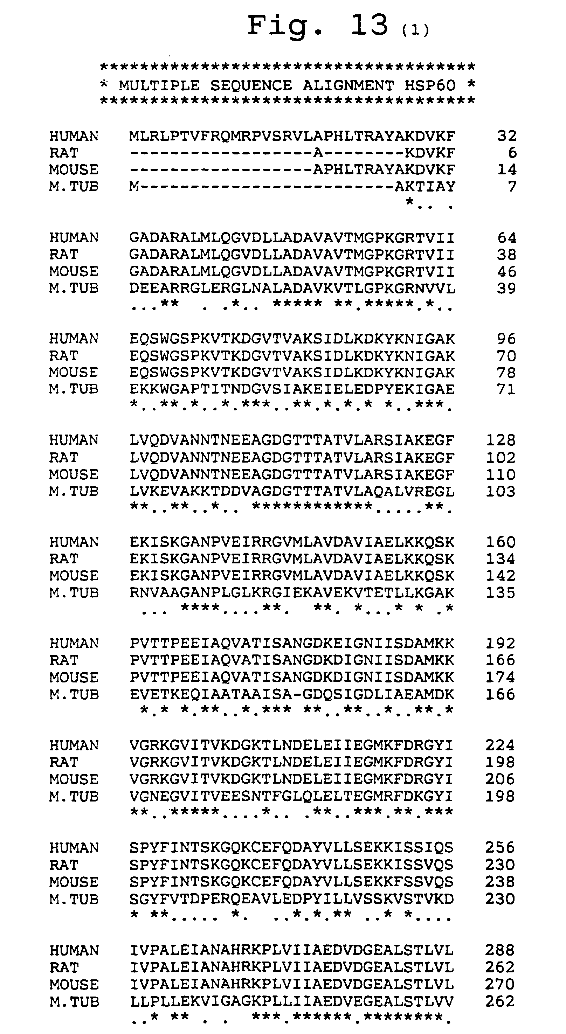

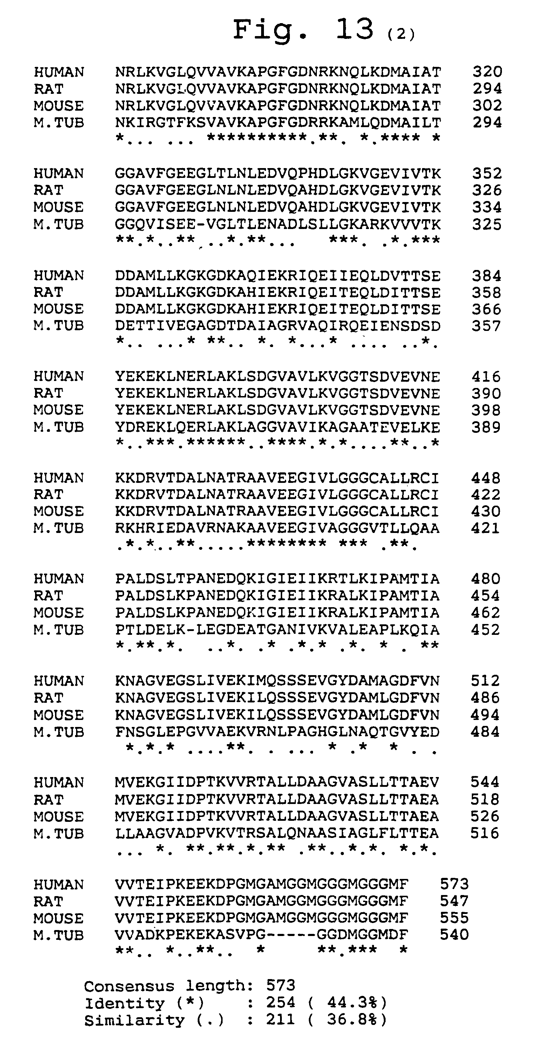

This method for developing the present peptides can be exemplified for the highly conserved enzyme glyceraldehyde-3-phosphate dehydrogenase (G3-PD):
1) Protein sequence alignments.
   The aminoacid sequence of the microbial protein to be used is aligned first with the sequence of the mammalian (e.g. rat) homologue as shown for the G3-PD protein in SEQ ID No. 2 and 3 and Fig. 14.
2) Selection of epitopes according to homology criteria.
   Regions are searched where the bacterial sequence contains at least 4 consecutive aminoacids which are identical to the corresponding residues at the same relative position within the mammalian protein sequence (e.g. regions 2-5, 7-14, 76-79, 92-97, 116-121, 130-134, 147-158, etc. up to 321-325, see SEQ ID No. 2 and 3 and Fig. 14). Then, synthetic peptides are prepared identical to the microbial sequence (5-30 aminoacids), each peptide overlapping with at least 4 aminoacids of these identical regions (for instance peptides 50-79, 92-121, 130-134, 167-178, 321-333).
3) Determination of T cell epitope qualities of selected sequences.
   Synthetic peptides thus selected, are used to identify individual epitopes in the bacterial G3-PD protein molecule for instance by the following procedures.
   a) After immunisations with the whole bacterial G3-PD protein, T cell responses to the peptides are monitored, to determine whether individual T cell epitopes are contained within the selected peptides. Additionally, since immunity to conserved bacterial proteins is likely to be pre-existent, due to priming with bacterial antigens, epitopes can be detected by direct in vitro screening of secondary T cell responses to the peptides, without prior immunisation.
   b) Alternatively, by immunisations with these peptides their capacity to induce sequence specific T cell responses to the microbial G3-PD protein, will reveal their T cell epitope qualities.
   A priori prediction of possible epitopes can be made on the basis of known MHC (HLA) binding motifs.
   The in vitro method for screening in all cases can be performed by the use of standard lymphocyte proliferation assays. Alternatively, other signs of T cell activation can be measured, such as production of cytokines, Ca2+ fluxes, cell body enlargement and increased or changed cell surface marker expression.
   Definition of T cell epitopes can be done in patients, healthy individuals and/or vaccinated/specifically immunized individuals. These individuals are preferably HLA-typed.
4) Determination of the capacity of selected epitopes to induce T cells cross-reactive with homologous self-proteins.
   T cells activated in vitro with the defined microbial epitopes, can then be restimulated with the homologous self protein (in the rat model the rat G3-PD, either as a recombinant protein or purified from stressed cells or tissue or as elevated levels of MHC-peptide complexes on stressed antigen presenting cells) or the homologous peptide. Any sign of activation (see under 3) can be taken as an indication of cross-reactivity of the microbial epitope with the self protein. Initial testing can be carried out with a synthetic peptide based on the homologous sequence of the self protein, but final proof for cross-reactivity with the protein itself, either in isolated form or expressed on cells, should be obtained, in order to exclude cryptic epitopes.

The peptides to be used according to invention can e.g. correspond to at least a part of a T cell epitope of the heat shock protein hsp65 of Mycobacterium tuberculosis. For Lewis rats e.g., T cell epitopes of hsp65 are located in the regions approximately defined by aminoacid residues 91-100, 176-190, 216-225, 226-235, 256-265, 386-400, 396-405, 446-455 and 511-520 respectively, of the mycobacterial hsp65 sequence as depicted in SEQ ID No. 1. The existence of T cell epitopes other than those indicated above is not excluded.

It was found that immunisation of rats with a peptide corresponding to sequence 256-270 of SEQ ID No.1 induced strong protection against induction, seven days later, of adjuvant arthritis (AA). Immunisation with a peptide corresponding to sequence 86-100 of SEQ ID No.1 induced moderate protection, whereas immunisation with peptides corresponding to the other epitopes produces little or no protection against adjuvant arthritis.

The T cell line H.52, originally generated from hsp65 immunised rats and specific for epitope 256-265 also showed a protective effect on AA development when injected i.v. at the time of administration of Mycobacterium tuberculosis.

It is concluded from this that protective peptides in microbial hsp65 are located at positions where at least 5 aminoacids are in the same relative position as the same aminoacids in a T cell epitope of microbial hsp65 that contains at least 4 consecutive aminoacids which are identical with the corresponding mammalian hsp60 aminoacids. Mammalian hsp includes human, rat and mouse hsp. The human, rat, mouse and mycobacterial hsp60/hsp65 aminoacid sequences are depicted in one letter code in Fig. 13. "Identical with the corresponding mammalian hsp60 aminoacids" is understood to mean that the aminoacid in question is identical with the aminoacid which is in the same position in either human, rat, or mouse hsp60.

The peptides are especially those having 5 aminoacids which are in the same relative position as the same aminoacids in one of the sequences 81-100 and 241-270 of SEQ ID No. 1, more particularly having at least 5 aminoacids which are in the same relative position as the same aminoacids in one of the sequences 84-95 and 256-265 of SEQ ID No. 1. With preference, the peptides comprise at least 6, or even 7, aminoacids with the same relative positions as those in the hsp65 T cell epitopes. Those epitopes are especially those which have at least 4 consecutive aminoacids which are identical with the corresponding mammalian hsp60 aminoacids. Examples of suitable peptide comprise the sequences [Ala Thr Val Leu Ala], [Ala Leu Ser Thr Leu] and [Leu Ser Thr Leu Val]. In particular, the peptide comprises 5-30 aminoacids of the amino acid sequence of hsp65. The hsp65 aminoacids may be coupled to other sequences, such as spacer sequences or fused peptide sequences.

The peptides are suitable for treatment of and protection against inflammatory diseases, including autoimmune diseases, such as diabetes, arthritic diseases, atherosclerosis, multiple sclerosis, and myasthenia gravis. Also inflammatory processes leading to transplant rejection may be suppressed by the peptides.

The invention also concerns the use of peptide analogues which exhibit the immunological properties of the peptides described above, but which contain one or more chemical modifications. Such peptide analogues, also referred to as peptidomimetics, can e.g. consist of units corresponding to the aminoacid residues of the peptides described above, wherein essentially the same side groups are present, but wherein the backbone contains modifications such as substitution of an amide group (CO-NH) by another group such as CH=CH, CO-O, CO-CH₂ or CH₂-CH₂. Other modifications, such as substitutions of an aminoacid by a similar natural, or non-natural aminoacid are also envisaged. In this respect, "similar" means having about the same size, charge and polarity; thus the aliphatic amino acids alanine, valine, norvaline, leucine, isoleucine, norleucine and methionine can be considered as similar; likewise the basic to neutral polar aminoacids such as lysine, arginine, ornithine, citrulline, asparagine and glutamine are similar for the present purpose; the same applies to the acidic to neutral polar aminoacids like asparagine, aspartate, glutamine, glutamate, serine, homoserine and threonine.

The peptides described above may be used as such, or may be coupled to a sequence which enhances their antigenicity or immunogenicity. Such sequences may include parts of toxoids or immunoglobulins. The peptides may also be used as complexes with MHC molecules and/or incorporated in liposomes. The peptides may also be covalently coupled to other molecules or whole cells as a vector for immunostimulation. The peptides may be in the form of monomers, dimers or multimers.

The invention also relates to the use of nucleotide sequences encoding the peptides described above. Such nucleotide sequences may comprise 15 or more nucleotides encoding a region corresponding to a T cell epitope of the stress protein. The nucleotide sequence of stress proteins is known or can be determined by conventional means. As an example, the nucleotide sequence of Mycobacterium tuberculosis gene encoding hsp65 is depicted in SEQ ID No. 1. Examples of suitable nucleotide sequences include the sequences 271-285, 766-780 and 769-783 of SEQ ID No. 1 and degenerate sequences and sequences hybridising with these sequences. The nucleotide sequences according to the invention can be used as such or incorporated in vector sequences, as a vaccine material for producing immunising peptides in vivo ("naked DNA approach").

An expression system capable of expressing a peptide described above, which system contains a nucleotide sequence corresponding to a part of the sequence encoding the stress protein, under the operational control of promoter sequences and other regulatory sequences can be present in a vector organism or in a cell, especially a eukaryotic cell, and can be used to produce the peptides used according to the invention on a larger scale.

The invention also concerns the use of antibodies, in particular monoclonal antibodies directed at the peptides described above. The antibodies can be produced using known methods, e.g. by hybridoma technology. The antibodies may be used as a passive vaccine or as a diagnostic tool.

The pharmaceutical composition suitable for protection against or treatment of an inflammatory disease, including autoimmune diseases, diabetes, arthritic diseases, atherosclerosis, multiple sclerosis and myasthenia gravis, may be in the form of a vaccine; it can then also contain a conventional adjuvant, such as Alu adjuvants, Iscoms, Freund's complete or incomplete adjuvant or other adjuvant, and/or carrier materials and other additives.

The composition may in particular be in the form of a medicine suitable for curing developing or developed inflammatory diseases; it contains conventional additives and excipients. As a treatment composition, it may also contain an antibody against the peptides described above. The vaccines and medicaments according to the invention may e.g. be in a form suitable for parenteral, oral or nasal administration.

The invention also relates to diagnostic methods based on the peptides described above, or the corresponding antibodies or nucleotide sequences (probes) as they can be used to measure the specific expression of peptide (epitope) sequences at the site of inflammation. Also considered are methods wherein peptides are used in assays measuring T cell proliferation or T cell cytokine production for diagnostic purposes.

### Abbreviations used in this description:

- AA:: adjuvant arthritis
- Ag:: antigen
- APC:: antigen presenting cell
- DDA:: dimethyl dioctadecyl ammonium bromide
- Dhbt:: 3-hydroxy-4-oxo-3,4-dihydro-1,2,3-benzotriazine
- FACS:: fluorescence activated cell sorter
- FCS:: foetal calf serum
- FITC:: fluorescein isothiocyanate
- Fmoc:: 9-fluorenylmethoxycarbonyl
- Hobt:: N-hydroxybenzotriazole
- hsp60:: mammalian 60 kDa heat shock protein
- hsp65:: mycobacterial 65 kDa heat shock protein
- IFA:: incomplete Freund's adjuvant
- JCA:: juvenile chronic arthritis
- 2-ME:: 2-mercaptoethanol
- MHC:: major histocompatibility complex
- Mt:: heat-killed Mycobacterium tuberculosis
- PAL:: peptide amide linker (trademark)
- PBS:: phosphate-buffered saline solution
- Pfp:: pentafluorophenyl
- PPD:: purified protein derivative of M.tuberculosis
- PLNC:: primed lymph node cells
- RA:: rheumatoid arthritis
- TCGF:: T cell growth factor
- TdR:: [³H] thymidine

### Materials and Methods

**Animals:** Male inbred Lewis rats (RT1 B¹ MHC haplotype) were obtained from the University of Limburg, Maastricht, The Netherlands. Rats were five to eight weeks old at the start of each experiment.

**Antigens and Adjuvants:** Heat-killed *Mycobacterium tuberculosis* strain H37Ra (Mt) was obtained from Difco. Purified protein derivative (PPD) of *M. tuberculosis* and purified recombinant hsp65 of *M.bovis* (which is identical to *M.tuberculosis* hsp65) were kindly provided by Dr. J.D.A. van Embden, National Institute of Public Health and Environmental Protection, Bilthoven, The Netherlands. Incomplete Freund's adjuvant (IFA, Difco) and dimethyl dioctadecyl ammonium bromide (DDA, Eastman Kodak, Rochester, NY, ref.18) were used as adjuvants. DDA was prepared as a 20 mg/ml suspension in PBS and sonicated to produce a gel which was mixed 1:1 with Ag solution prior to immunisation.

**Synthetic peptides:** Peptides were prepared by automated simultaneous multiple peptide synthesis (SMPS). The SMPS set-up was developed using a standard autosampler (Gilson 221) as described previously (19). Briefly, for the concurrent synthesis of peptides, standard Fmoc chemistry with Pfp-activated amino acids (Dhbt for serine and threonine) in a sixfold molar excess and Hobt as catalyst were employed. Peptides were obtained as C-terminal amides from 6 mg resin/peptide (0.33 meq/g PAL resin, Millipore). Two panels of peptides were synthesised, based on the sequences of Mt hsp65 (20) and rat hsp60 (21). Peptides were 15mers with ten amino acid overlap with each adjacent peptide (i.e residues 1-15, 6-20, 11-25 etc). Thus, each possible 11mer sequence of each protein was contained within a peptide.

Immunisations and primed lymph node populations: Rats were immunised with either Mt or hsp65. Mt was suspended at 5 mg/ml in IFA or DDA and 100 µl injected in each hind footpad (i.e. 500 µg/footpad, 1 mg/rat). Hsp65 (1 mg/ml in PBS) was mixed 1:1 with DDA and 100 µl injected (i.e. 50 µg/footpad, 100 µg/rat). Ten to 21 days later, draining popliteal lymph nodes were removed, disaggregated, washed three times and used as a source of primed lymph node cells (PLNC). In control experiments splenocytes and lymph nodes from unimmunised rats and PLNC from rats immunised with IFA or DDA/PBS alone were used.

**Immunisations with synthetic peptides and primed lymph node populations:** Rats were immunised with 50 µg of synthetic peptide in PBS/DDA in each hind footpad (i.e. 50 µg/footpad, 100 µg/rat). Ten days later, draining popliteal lymph nodes were removed, disaggregated, washed three times and used as a source of primed lymph node cells (PLNC). In some experiments, PLNC were derived as pooled inguinal and popliteal lymph nodes from AA rats 35-42 days post Mt immunisation.

**Tissue culture reagents:** Iscove's modification Dulbecco's medium (IMDM, Gibco) supplemented with 5% FCS, 2 mM L-glutamine, 100 U/ml penicillin, 100 µg/ml streptomycin (all from Gibco) and 5 x 10⁻⁵ M 2-ME was used as culture medium. Cell populations were washed in IMDM without supplements.

**T cell proliferation assays:** PLNC were cultured in triplicate in 200 µl flat bottom microtitre wells (Costar) at 2 x 10⁵ cells per well with or without antigen. In initial experiments PLNC were tested for responsiveness to individual peptides at concentrations of 5 and 20 µg/ml, and Mt, hsp65 and PPD over a range of concentrations. Concanavalin A (2 µg/ml) was used as a positive control for T cell proliferation. Cultures were incubated for 96 h at 37°C in a humidified atmosphere of 5% CO₂. Cultures were pulsed for the final 16 h with [³H] TdR (Amersham, U.K., 1 uCi/well) and TdR uptake measured using a liquid scintillation β counter.
Assays using T cell lines were performed as above using 2 x 10⁴ line cells per well with irradiated (30 Gy) syngeneic accessory cells (either 3 x 10⁵ splenocytes/well or 10⁶ thymocytes/well). Results are expressed as mean counts per minute (cpm) of triplicate cultures. In experiments where responses to Ag were low, responses were considered significant if the stimulation index (S.I. = mean cpm with Ag - mean cpm without Ag) was greater than two and Student's t tests gave p < 0.01.

**T cell lines:** T cell lines with specificity for mycobacterial hsp65 or peptides were generated by bulk stimulation of either hsp65-PLNC or Mt-PLNC. PLNC were cultured at 5 x 10⁶/ml in culture medium supplemented with 2% normal rat serum (NRS) in the presence of 10 µg/ml Ag. After three days viable cells were harvested using a ficoll-isopaque gradient and cultured for a further four days in culture medium + 5% FCS and 5% TCGF (Con A-activated rat spleen supernatant). Seven days after initial stimulation lines were restimulated with irradiated spleen accessory cells and 10 µg/ml Ag in culture medium + NRS. Lines were maintained ir. this seven day restimulation cycle.
Short term epitope-specific T cell lines were also generated from rats immunised with synthetic peptides. Peptide-PLNC were cultured as above in the presence of 10 µg/ml peptide.

**Monoclonal antibodies:** Anti-MHC monoclonal antibodies were added to proliferation assays to determine the MHC-restriction of responses to hsp65 and peptides. 0X6 (anti-RT1.B, class II), 0X17 (anti-RT1.D, class II), 0X18 (anti-RT1.A, class I) and UD15 (anti-chloramphenicol control antibody) were used. All antibodies were mouse IgG1.

**FACS Analysis:** FACS analysis was used to phenotype T cell lines. Cells were incubated with R73 (anti-αβ TCR), W3/25 (anti-CD4) or 0X8 (anti-CD8), all mouse IgG1 antibodies. Second step staining was with FITC-conjugated Goat anti-Mouse Ig (Becton Dickinson). Cells were analysed using a Becton Dickinson FACScan analyser.

**Induction and clinical assessment of experimental arthritis:** Arthritis was induced by a single intradermal injection in the base of the tail. AA was induced with 0.5 mg Mt suspended in 100 µl IFA. CP20961 arthritis was induced with 2 mg CP20961 in 100 µl light mineral oil (Sigma). Rats were examined daily for clinical signs of arthritis. Severity of arthritis was assessed by scoring each paw from zero to four based on degree of swelling, erythema and deformity of the joints. Thus the maximum possible arthritis score was 16.

**Modulation of arthritis with hsp65 peptides and epitope-specific T cell lines:** Synthetic peptides corresponding to eight T cell epitopes present in mycobacterial hsp65 were tested for protective effects on arthritis development. Rats were immunised with 100 µg of individual peptide seven days prior to arthritis induction. Peptides were immunised in each hind footpad in 10 mg/ml DDA (50 µl/pad). Control rats received only PBS/DDA. Epitope-specific T cell lines were also tested for protective activity by intravenous administration of lines at the time of arthritis induction. T cell lines were restimulated *in vitro* with irradiated spleen APC and specific peptide. Four days later T cell blasts were harvested by ficoll gradient, washed and applied to a second ficoll gradient to remove any contaminating APC. T cells were washed twice in wash medium and twice in PBS and finally suspended at 2.5x10⁷/ml in PBS. Immediately prior to injection of Mt, 200 µl (i.e. 5x10⁶) T cells were injected i.v. in the tail vein.

### Results

### Primed lymph node cell responses to Mt, PPD and hsp65.

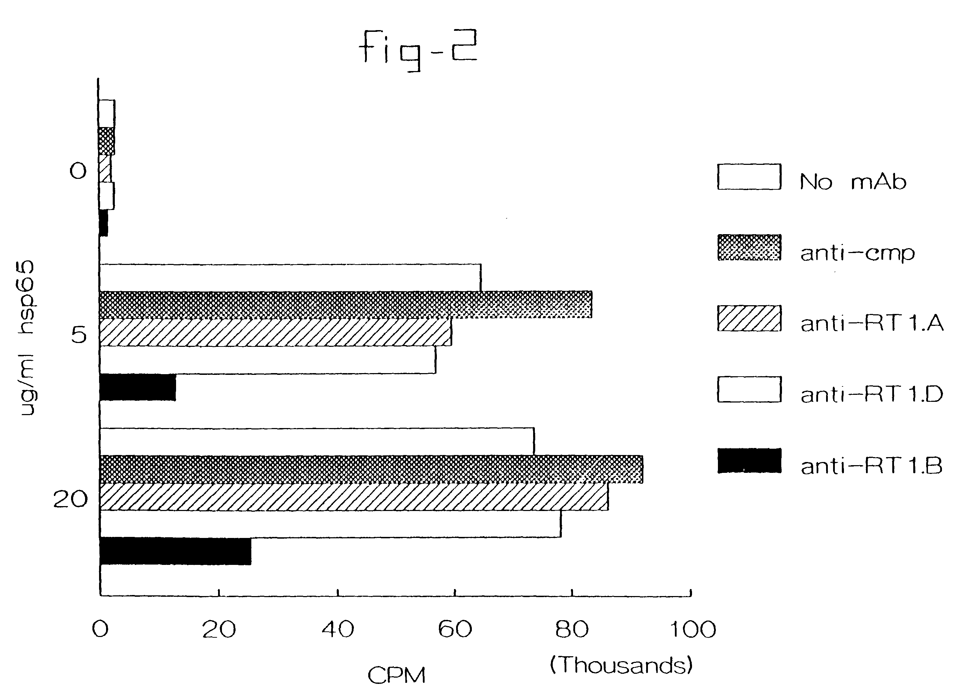

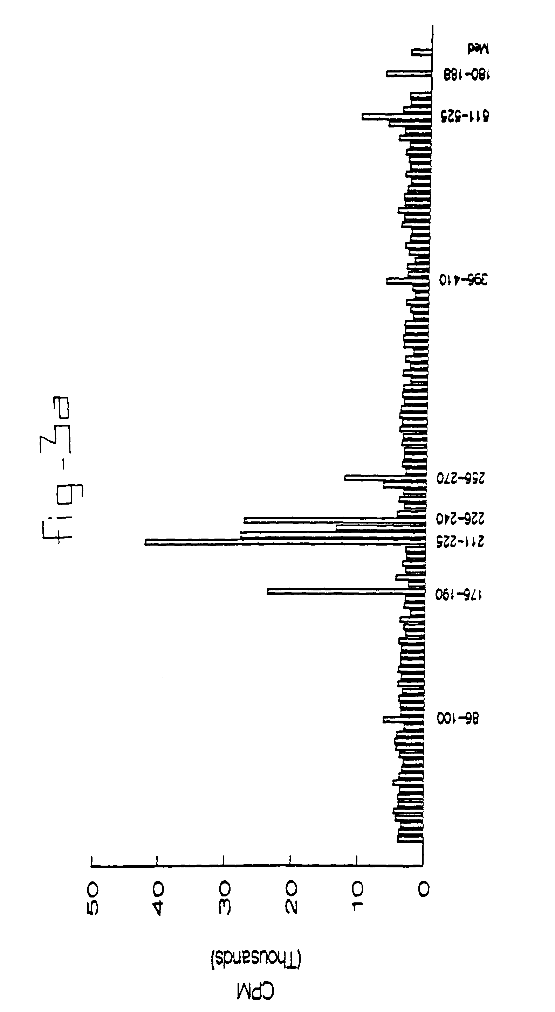

Immunisation with hsp65 primed for responsiveness to hsp65. PPD (which contains native hsp65) also induced proliferation indicating that immunisation with recombinant hsp65 activated T cells recognising native mycobacterial hsp65. Hsp65-PLNC showed increased responsiveness to Mt compared to DDA/PBS-PLNC. Hsp65-PLNC proliferative responses to hsp65 were effectively inhibited by addition of the 0X6 monoclonal antibody to cultures, indicating that the response was predominantly (if not totally) restricted to the RT1.B¹ MHC class II molecule (Fig. 2). No significant inhibition was seen using anti-RT1.D, anti-RT1.A or anti-chloramphenicol isotype control mAbs.

### Identification of hsp65 T cell epitopes following immunisation with hsp65

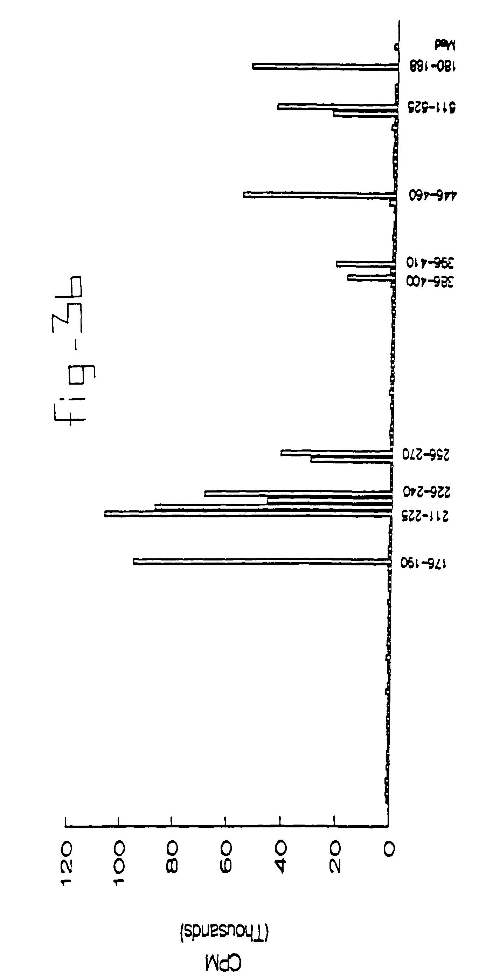

Hsp65-PLNC were analysed for responsiveness to a panel of peptides covering the entire sequence of hsp65 (Fig. 3a). Several peptides induced significant proliferation, suggesting the presence of seven epitopes. In terms of magnitude of response, three regions of the molecule (residues 176-190, 211-230 and 221-240) appeared to contain the "dominant" T cell epitopes, while regions 86-100. 251-270, 396-410 and 506-525 contained "subdominant" or minor epitopes. For four of these epitopes (211-230, 221-240, 251-270 and 506-525) responses to adjacent overlapping peptides were seen, suggesting that the core epitopes lay within 216-225, 226-235, 256-265 and 511-520 respectively.

Peptide 176-190 contained the AA-associated epitope 180-188. A synthetic peptide 180-188 was also tested. It was found to induce responses, but at a lower level than the longer 176-190 peptide, indicating that the minimal epitope provides less efficient stimulation at the PLNC level.

PLNC responses were tested at 10, 14 and 21 days post immunisation. Responses were strongest at day ten and declined with time. The pattern of dominance remained constant and no "new" responses to different peptides were observed at day 21. Initial experiments used pooled PLNC from several immunised rats. Experiments testing the responsiveness of individual rats showed no variation between rats in the pattern of responsiveness. Peptides that stimulated hsp65-PLNC were also tested on unprimed splenocytes and DDA/PBS-PLNC and were found to have no stimulatory activity, indicating that immunisation with hsp65 was required to prime for the peptide induced responses.

Hsp65-PLNC were also tested for responses to a panel of peptides covering the entire sequence of rat hsp60. None of the rat hsp60 peptides induced significant responses in hsp65-PLNC tested at 10 or 21 days post immunisation.

To confirm the presence of the hsp65 T cell epitopes identified at the PLNC level, short term T cell lines were established from ten day hsp65-PLNC by bulk stimulation with hsp65. Seven days after the first restimulation, these lines were tested against the entire panel of hsp65 peptides (Fig. 3b). The response pattern obtained was similar to that of the original PLNC populations. The three dominant epitopes, 176-190, 216-225 and 226-235, all induced strong responses, and epitopes 256-265, 396-410 and 511-520 were also recognised. Two peptides that failed to stimulate hsp65-PLNC (386-400 and 446-460) did stimulate the hsp65-specific lines.

### T cell responses to hsp65 epitopes following immunisation with whole M. tuberculosis.

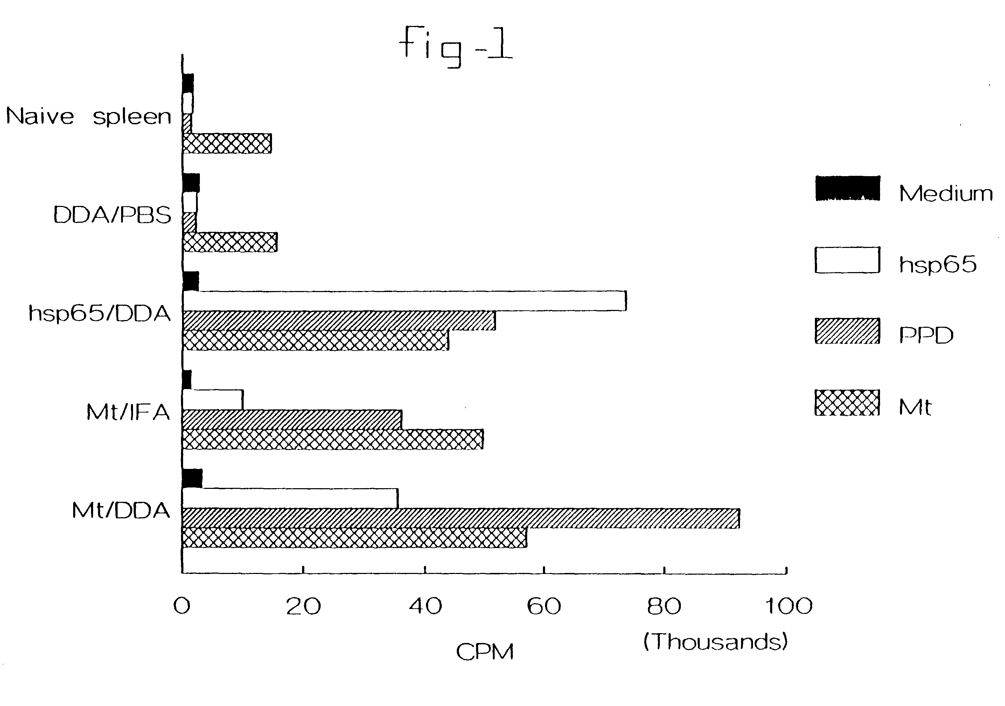

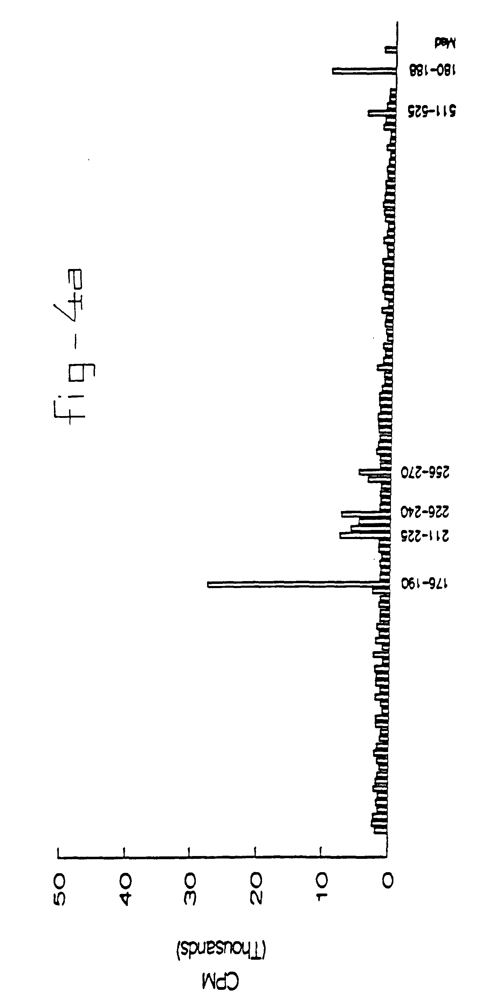

Mt-PLNC were tested for responses to hsp65 peptides, to determine whether immunisation with whole Mt could prime for responsiveness to the hsp65 epitopes described above. Immunisation with Mt/IFA (i.e. the AA-inducing protocol) consistently failed to induce significant responses to hsp65 peptides. As Mt/IFA immunisation induced only low level reactivity to hsp65 (Fig. 1), PLNC from rats immunised with Mt mixed with DDA were tested. This protocol, (using 500 µg or 1 mg of Mt per rat) produced PLNC showing increased reactivity to hsp65, and significant responses to hsp65 peptides (Figs. 1 and 4a). The response pattern differed from that obtained using hsp65-PLNC. Epitope 176-190 appeared to be dominant, epitopes 216-225, 226-235, 256-265 and 511-520 were minor and responses to epitopes 86-105 and 396-410 absent (Fig. 4a).

### Analysis of T cell lines specific for defined hsp65 epitopes

T cell lines were generated by restimulation of hsp65-PLNC with individual synthetic peptides. For epitopes where responses had been detected to two overlapping peptides, the line was generated using the peptide that induced strongest proliferation of hsp65-PLNC (e.g. for epitope 216-225, peptide 211-225 was used). This resulted in eight T cell lines. Table I summarises the response patterns to each epitope and names each peptide-specific T cell line. No lines specific for peptide 386-400 were established.

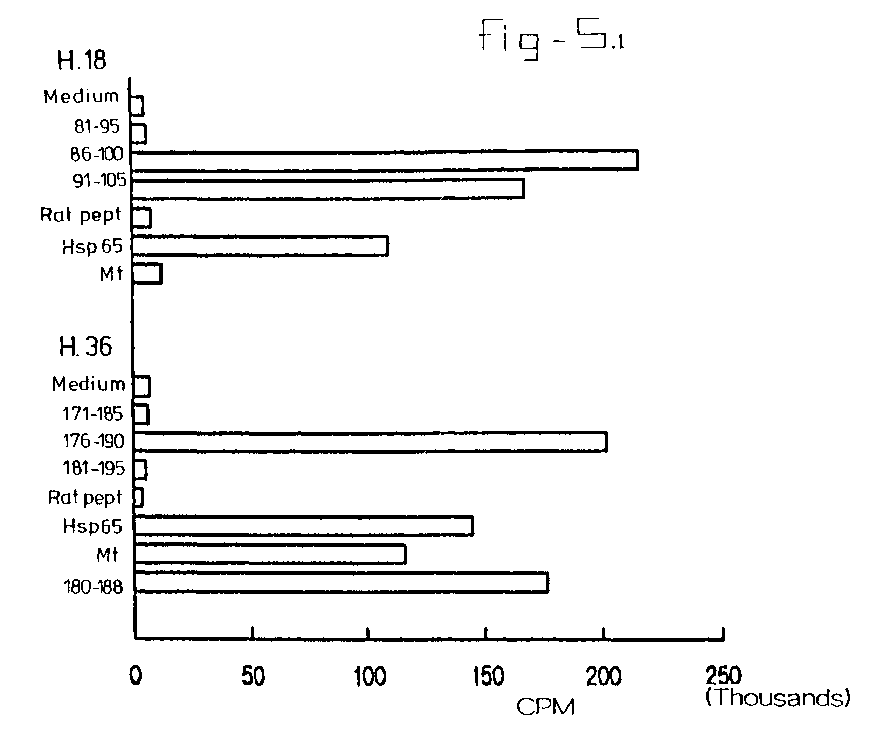

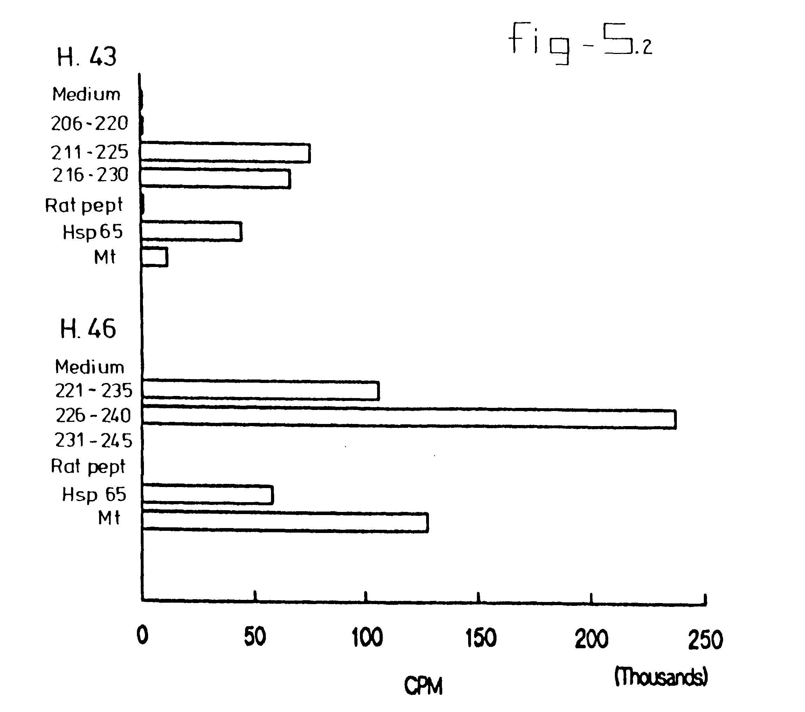

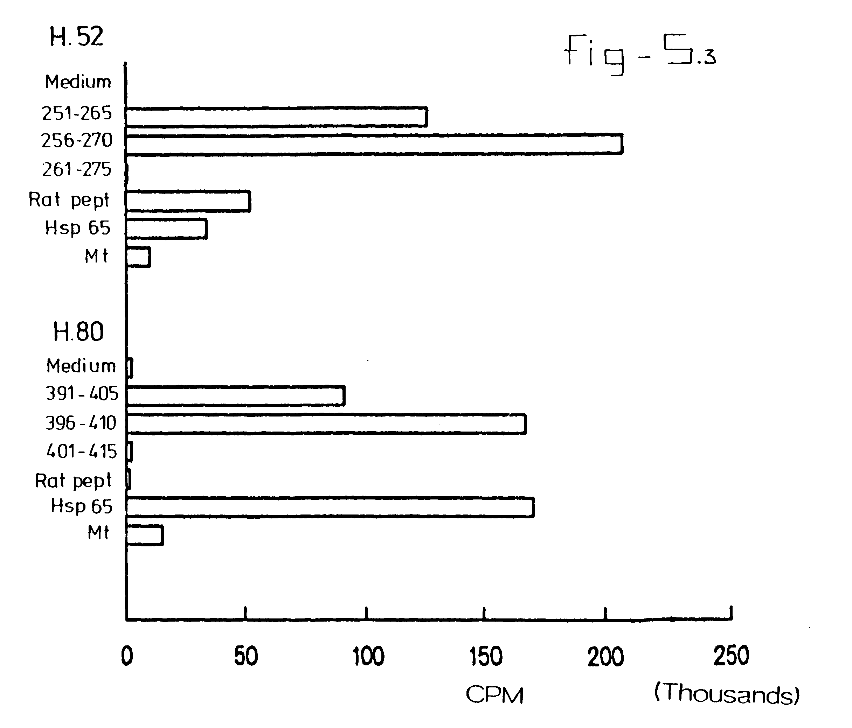

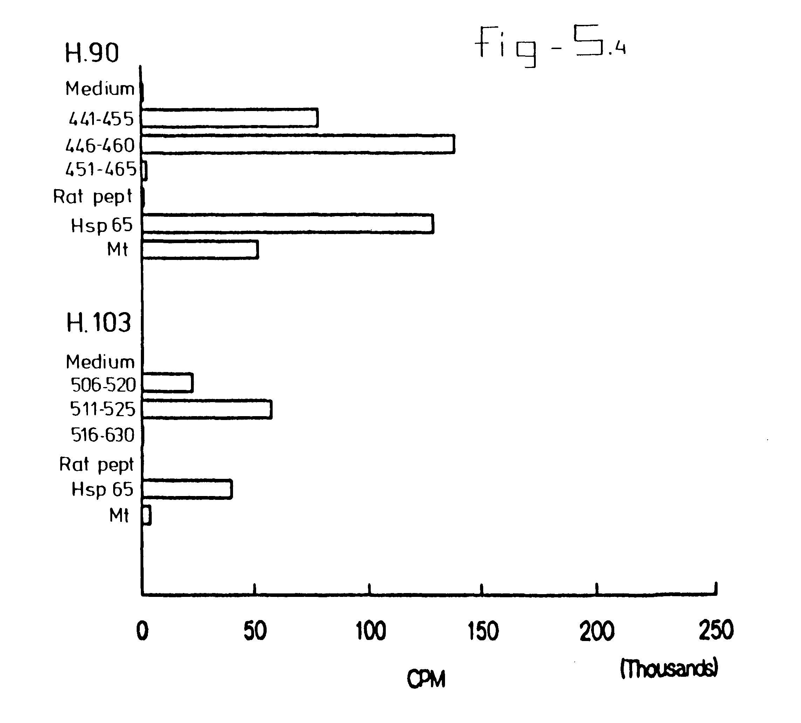

After at least four in vitro restimulations each line was tested for responsiveness to specific peptide, hsp65, Mt and PPD. To test for cross-reactivity with self hsp60, each line was also tested against the corresponding rat hsp60 peptide (Fig. 5).

All lines responded to hsp65 and their specific peptides and, in all but one instance, an overlapping peptide. Between residues 211 and 235 four overlapping peptides were stimulatory. Thus it was possible that four separate epitopes were present in this region. However, T cell lines generated against peptides 216-230 and 221-235 responded more strongly to peptides 211-225 and 226-240 respectively, indicating the presence of only two epitopes: 216-225 and 226-235 as described above. Line H.36, specific for peptide 176-190 also responded to a peptide including residues 180-188, previously described to be recognised by the arthritogenic T cell clone A2b, indicating that these two lines recognise the same core epitope.

Line H.52, specific for residues 256-265, showed clear responses to the homologous peptide of rat hsp60. Therefore, while no responses to rat hsp60 peptides could be detected using hsp65-PLNC or the hsp65-specific lines, cross-reactive T cell recognition could be demonstrated using this epitope-specific cell line. The mycobacterial peptide induced a five to ten fold higher level of proliferation than the rat peptide. This might explain why the rat peptide was not recognised at the PLNC level as responses to the mycobacterial peptide were minor and any decrease in stimulatory activity could result in responses below the detectable level. The core epitope recognised by H.52 contains only three residues which differ from the rat hsp60 sequence, situated at the C terminal end of the epitope (see Table I). All other T cell lines failed to respond to rat hsp60 peptides. Thus, of the nine T cell epitopes in mycobacterial hsp65 identified by this study, only one showed cross-reactivity with rat hsp60.

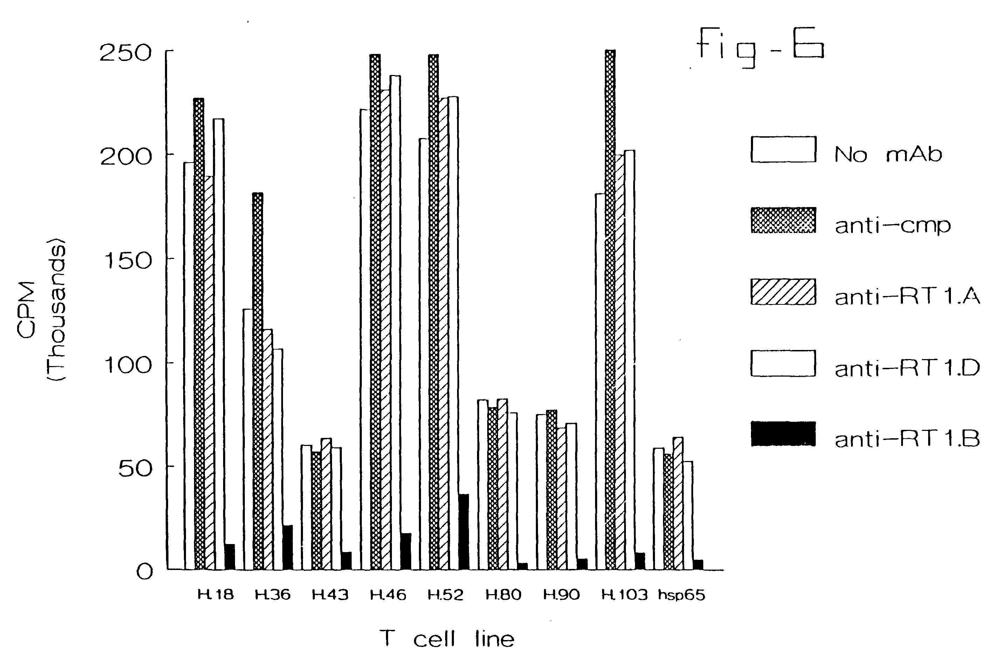

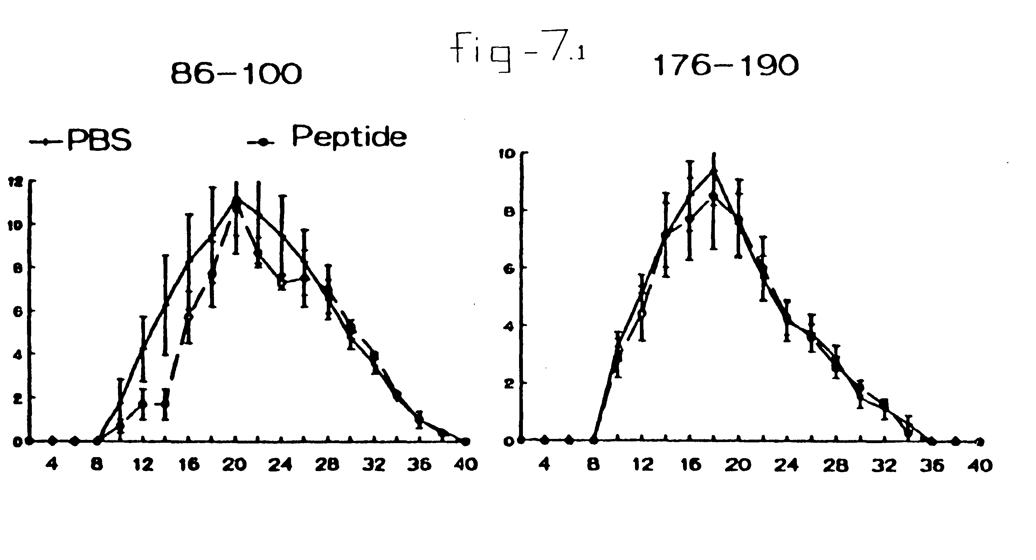

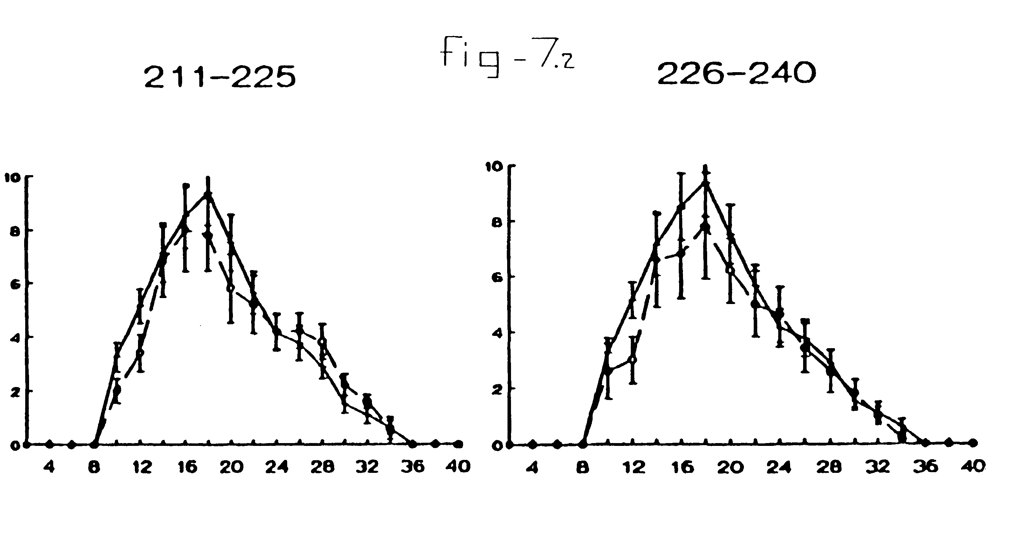

All peptide-specific or hsp65-specific T cell lines were analysed for T cell phenotype and MHC-restriction. FACS analysis showed that all T cell lines were αβ TCR⁺ CD4⁺ CD8⁻. MHC-restriction was determined by assessing the ability of anti-MHC monoclonal antibodies (10 µg/ml) to inhibit hsp65 or peptide (1 µg/ml) induced responses in proliferation assays. Addition of anti-RT1.B reduced proliferation of all lines by greater than 70%, while anti-RT1.D or anti-RT1.A had no significant effect (Fig. 6). Thus all lines were RT1 B¹-restricted.

### Immunisation with hsp65 peptides primes for epitope-specific T cell responses

To determine whether immunisation of rats with synthetic peptides was effective at priming for T cell reactivity to hsp65 epitopes, in vitro proliferative responses following immunisation with 100 µg of peptide each containing individual T cell epitopes were examined. PLNC isolated ten days after immunisation were tested for response to the immunising peptide, overlapping peptides and to hsp65 (Table II). PLNC responses were observed after immunisation with seven of the nine peptides. Responses were not seen after immunisation with peptides 386-400 and 511-525.

Bulk stimulation generated T cell lines to eight of the nine peptides tested. These eight lines were tested for specificity after four *in vitro* restimulations (Table II). All responded to the immunising peptide and, to some extent, to hsp65. Also, the lines were tested for responsiveness to overlapping peptides and, in all but one case, showed identical response patterns to T cell lines generated previously against the same epitopes after immunisation with whole hsp65. These findings suggest that T cells activated *in vivo* by immunisation with hsp65 or synthetic peptides recognise the same core epitopes.

A T cell line specific for the 86-100 peptide generated from rats immunised with hsp65 responded to peptides 86-100 and 91-105 (i.e. it recognised a core epitope of 91-100). The T cell line generated following immunisation with the 86-100 peptide also responded to peptides 81-95 and 91-105. This suggested the presence of two T cell populations, one recognising residues 86-95, the other recognising residues 91-100. Interestingly, the sequences of mycobacterial hsp65 and rat hsp60 covering residues 86-95 are identical. Accordingly, the line proliferated in response to peptides corresponding to rat hsp60 81-95 and 86-100. Thus immunisation with the hsp65 86-100 peptides could prime for responsiveness to an epitope in self hsp60.

Line H.52 (generated from hsp65-immunised rats and recognising epitope 256-265) also responded to the highly homologous rat hsp60 256-270 peptide (see aoove). Accordingly the 256-270 specific T cell line derived from peptide immunised rats also responded to rat 256-270 (Table II). This line also showed an increased "autoreactive" response to syngeneic APC which had been heat-shocked (one hour at 42°C prior to irradiation) in comparison control APC cultured at 37°C (Table III). This suggested that increased expression of endogenous hsp60 by APC results in presentation of this cross-reactive epitope in association with MHC for T cell recognition.

Initial testing of peptides as described in the present application in humans has shown that both peptides 256-270 and 86-100 are capable of inducing T cell proliferative responses in some autoimmune disease patients. Thus, already the rat-defined T cell epitopes themselves could be used for therapeutical intervention in some individuals (certain HLA types).

### Analysis of the ability of hsp65 peptides to vaccinate against arthritis

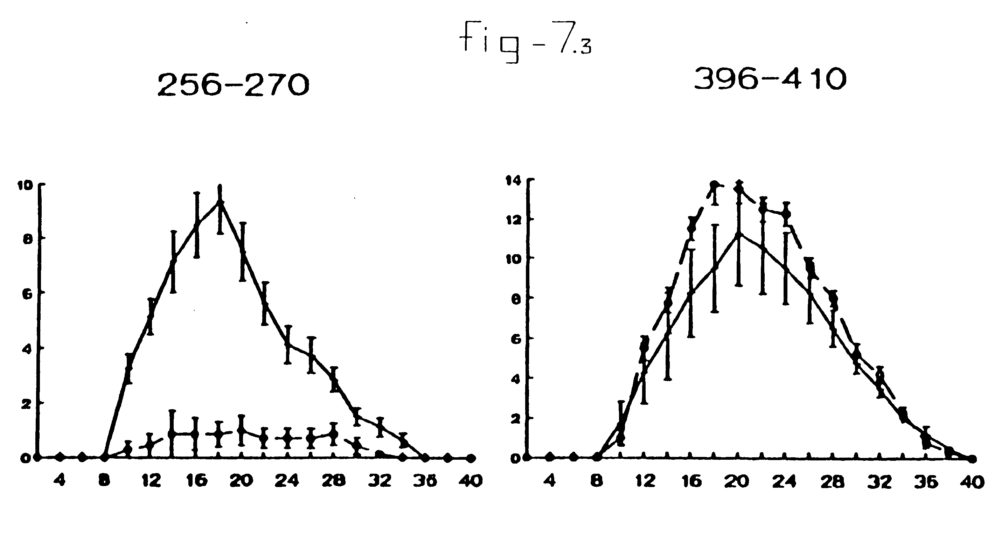

The effects of preimmunisation with synthetic peptides containing individual epitopes on the development of AA were analysed (Fig. 7). The eight peptides which primed for epitope specific T cells were tested. Rats were immunised with 100 µg of peptide 7 days prior to AA-induction with Mt.

Preimmunisation with peptide 256-270 resulted in clear protective effects against AA development. The mean maximum arthritis score after 256-270 preimmunisation was 1.7 (24 rats in five separate experiments) compared to mean maximum score of 11.5 for control rats preimmunised with PBS. Of the 24 rats preimmunised with 256-270, twelve did develop clinical signs of arthritis, which were milder than those developed by control rats. Also, whereas control rats suffered permanent joint deformities that persisted after the initial arthritis had subsided, the 256-270 preimmunised rats that developed AA were remarkably free of permanent deformities. Preimmunisation with the other peptides containing hsp65 T cell epitopes had no significant effects on the onset of AA, except for a moderate effect of peptide 86-100.

PLNC from preimmunised rats were tested for responsiveness to hsp65 epitopes 42 days after Mt administration (Fig. 8). PLNC from rats preimmunised with PBS/DDA alone showed significant responses to peptide 176-190 (containing the AA-associated 180-188 epitope) but not to any other of the defined hsp65 epitopes. PLNC from rats preimmunised with individual hsp65 peptides all showed responses to the immunising peptide, although the level of proliferation varied. Thus, whereas preimmunisation with peptides 211-225 and 446-460 induced strong responses, preimmunisation with peptide 256-270 (which protected against AA) induced marginal PLNC responses. Responses to peptide 176-190 containing the AA-associated epitope were found in all PLNC populations (and enhanced after 176-190 preimmunisation) with one exception. PLNC from rats protected from AA by preimmunisation with peptide 256-270 showed a total lack of response to 176-190.

### Administration of a T cell line specific for the cross-reactive 256-265 epitope confers protection against AA

The effects of epitope-specific T cell lines on AA were tested (Fig. 9). T cell lines were administered to rats at the same time as Mt for AA induction (5x10⁶ T cells per rat injected i.v. in a tail vein). In two experiments, administration of line H.52 recognising the 256-265 epitope clearly reduced the severity of AA compared to control animals receiving PBS. The T cell lines H.36, H.43 and H.46 (specific for epitopes 180-188, 216-225 and 226-235 respectively) were also tested and had no significant effects on AA development.

Line H.46 was restimulated with specific peptide (226-240) in the presence of 10 µg/ml peptide 256-270, in order to ensure that the protective effect of line H.52 was not the result of administration of residual 256-270 peptide carried over from the in vitro restimulation of the line. Administration of the resulting 226-235 specific T cell blasts failed to protect against AA.

### Preimmunisation with peptide 256-270 also vaccinates against CP20961-induced arthritis

The only hsp65 peptide which protected strongly against AA was found to be 256-270, which induced T cell reactivity against the corresponding sequence of rat hsp60. This finding suggested that self-reactive T cells account for hsp65-induced protection against arthritis. This mechanism would then be expected to protect against arthritis induced by other compounds and not be dependent on the use of mycobacteria. Therefore the capacity of peptide 256-270 to induce protection in the CP20961-induced model was tested. As CP20961 is a lipoidal amine, there is no possibility of this arthritogenic preparation containing an antigenic component with potential cross-reactivity with hsp65 256-270. Preimmunisation with hsp65 256-270 strongly protected rats against CP20961-induced arthritis, whereas a control peptide (hsp65 211-225) did not (Fig. 10). Thus preimmunisation with hsp65 256-270 can protect against AA and CP20961-induced arthritis, presumably via a mechanism which is not dependent on the administration of mycobacteria as a component of the arthritogenic inoculation.

### Rat hsp60 (256-270) fails to vaccinate against arthritis

Since preimmunisation with hsp65 256-270 provided protection against AA and T cells specific for this peptide also responded to the highly homologous peptide based on the rat hsp60 256-270 sequence, the rat peptide was tested for similar protective effects (Fig. 11). Protection was observed after preimmunisation with mycobacterial hsp65 256-270, but not with rat hsp60 256-270. An explanation for this discrepancy may be that immunisation with rat hsp60 256-270 does not prime for appropriate T cell responses. To investigate this we generated peptide-specific T cell line from rat 256-270 (R.256-270) immunised PLNC and compared responses of this line with that of a line from mycobacterial 256-270 (M.256-270) immunised PLNC (Fig. 12). The line generated against M.256-270 responded to peptides representing M.256-270, M.251-265 and a shorter "core" peptide M.256-265, and cross-reacted with R.256-270 and (weakly) R.256-265. In contrast, the line generated against R.256-270 responded to R.256-270, but not to M.256-270 or to R.256-265 and M.256-265. This line also showed a high "autoreactive" response to APC alone, suggesting that the APC were already expressing the self hsp60 epitope.

Thus immunisation with M.256-270 primed for T cell responses that cross-reacted with R.256-270, but immunisation with R.256-270 primed for rat-specific T cell responses that did not cross-react with M.256-270. The fact that the R.256-270 specific line did not respond to R.251-265 or R.256-265 suggests that one or more of the five C-terminal residues of R.256-270 is important for stimulation of this line. None of these five residues share identity with the mycobacterial sequence, presumably accounting for the lack of responsiveness to M.256-270.

Furthermore, using anti-MHC mAbs, the T cell lines generated against M.256-270 and R.256-270 were found to have different MHC-restrictions. Peptide induced proliferation of the M.256-270 specific line was inhibited by the 0X6 anti-RT1.B mAb, whereas the R.256-270 specific line was inhibited by the 0X17 anti-RT1.D mAb.

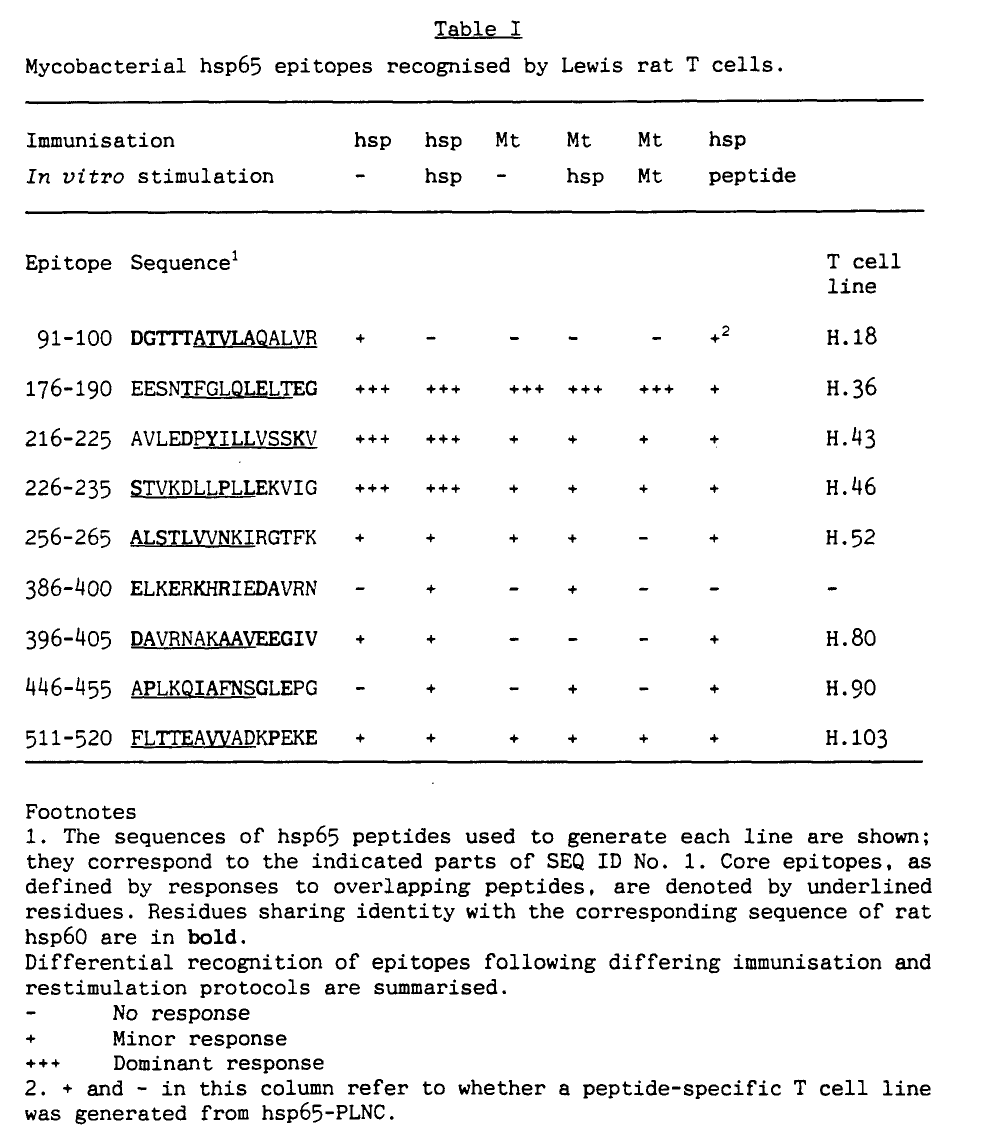

### Table II

T cell responses by immunisation with hsp65 peptides.
Rats were immunised with synthetic peptides containing individual hsp65 epitopes (100 µg peptide/DDA per rat). Ten days later PLNC were isolated and tested for responses to overlapping peptides (20 µg/ml). Peptide-specific T cell lines were generated by bulk in vitro stimulation of PLNC with immunising peptide. Lines were tested for responses to overlapping peptides (10 µg/ml). All PLNC and T cell lines showed significant responses to 20 µg/ml hsp65. Results are expressed as mean cpm of triplicate cultures. All SEM were less than 20%.

**Table II**

| Immunising peptide | *In vitro* peptide | CPM | | Response after hsp65 immunisation¹ |
|---|---|---|---|---|
| | | PLNC | T Cell Line | |
| 86-100 | 0 | 2518 | 1127 | |
| | 81-95 | 15296 | 15785 | - |
| | 86-100 | 57925 | 49422 | + |
| | 91-105 | 1999 | 18444 | + |
| | Rat 86-100 | 6196 | 25535 | - |
| | | | | |
| 176-190 | 0 | 1784 | 1255 | |
| | 171-185 | 3082 | 1640 | - |
| | 176-190 | 57707 | 31400 | + |
| | 181-195 | 1916 | 1653 | - |
| | 180-188 | 7859 | 23275 | + |
| | | | | |
| 211-225 | 0 | 986 | 1479 | |
| | 206-220 | 645 | 988 | - |
| | 211-225 | 39765 | 121978 | + |
| | 216-230 | 11117 | 102341 | + |
| | | | | |
| 226-240 | 0 | 1286 | 882 | |
| | 221-235 | 7381 | 100306 | + |
| | 226-240 | 22761 | 152071 | + |
| | 231-245 | 1655 | 862 | - |
| | | | | |
| 256-270 | 0 | 2448 | 762 | |
| | 251-265 | 6391 | 66063 | + |
| | 256-270 | 16423 | 69037 | + |
| | 261-275 | 2553 | 646 | - |
| | Rat 256-270 | 4152 | 14987 | + |
| 396-410 | 0 | 3399 | 2095 | |
| | 391-405 | 2280 | 39335 | + |
| | 396-410 | 8477 | 88916 | + |
| | 401-415 | 3211 | 2011 | - |
| 446-460 | 0 | 1928 | 1028 | |
| | 441-455 | 4715 | 17805 | + |
| | 446-460 | 22918 | 38324 | + |
| | 451-465 | 2130 | 1283 | - |
| 511-525 | 0 | 2804 | 537 | |
| | 506-520 | 2754 | 61293 | + |
| | 511-525 | 3164 | 129373 | + |
| | 516-530 | 2821 | 1231 | - |

| | | | | |
|---|---|---|---|---|
| ¹. As determined using T cell lines generated from hsp65-immunised rats. | | | | |

**Table III**

| | | |
|---|---|---|
| Stimulation of hsp65(256-270) specific T cells by heat-shocked APC. T cell lines (2 x 10⁴/well) were cultured with APC (2 x 10⁵/well) that had been cultured for one hour at either 37°C or 42°C prior to irradiation. Cells were cultured with or without specific peptide as Ag (10µg/ml). Line H.46, specific for the non-cross-reactive, mycobacterial hsp65 unique epitope 211-225 was used as a control. Results are expressed as mean cpm of triplicate cultures. All SEM were less than 20%. | | |

| T cell line Specificity | P.m52.1 256-265 | Line H.46 226-235 |
|---|---|---|
| T cells - APC | 44 | 22 |
| 37°C APC - Ag | 470 | 33 |
| + Myco. pept | 120744 | 162785 |
| + rat pept | 18061 | NT |
| 42°C APC - Ag | 15960 | 37 |
| + Myco. pept | 115626 | 150887 |
| + rat pept | 25842 | NT |

### Discussion

Immunisation of Lewis rats with heat-killed Mt in IFA induces AA (1), reported to be associated with T cell responses to residues 180-188 of mycobacterial hsp65 (5). Conversely, immunisation with hsp65 protects against subsequent attempts to induce AA by an as yet undefined T cell-mediated mechanism (5,6). Epitopes within hsp65 recognised by Lewis rat T cells were identified in this description. Responsiveness to these epitopes in T cell populations following immunisation with either Mt or hsp65 was compared.

Immunisation of rats with recombinant mycobacterial hsp65 primed for MHC class II (RT1.B¹)-restricted recognition of hsp65. Analysis of hsp65-PLNC responses to overlapping peptides covering the entire hsp65 sequence identified seven T cell epitopes. Following a single in vitro restimulation with hsp65, two further epitopes were identified. Thus Lewis rat T cells recognise nine epitopes in mycobacterial hsp65.

Immunisation with Mt suspended in IFA (as used in the AA-inducing protocol) did not prime for PLNC responses to hsp65 peptides. However, Mt/DDA immunisation did induce significant responses to hsp65 peptides. Significantly, AA only develops following Mt/IFA immunisation and not after Mt/DDA immunisation (S.M.A, unpublished observations). Thus the enhanced activation of hsp65-specific T cells when DDA is used as adjuvant might account for this difference. T cells from Mt/IFA and Mt/DDA immunised rats showed identical response patterns after *in vitro* restimulation with hsp65 indicating that Mt/IFA does prime for T cell recognition of hsp65, but at a relatively low level.

Patterns of dominance of hsp65 epitopes differed following immunisation with hsp65 or whole Mt. Hsp65 immunisation resulted in three co-dominant epitopes: 176-190, 216-225 and 226-235. After Mt immunisation, epitope 176-190 appeared dominant with epitopes 216-225 and 226-235 being minor along with 256-265 and 511-520. The dominance of the 176-190 epitope was even more marked when Mt-PLNC were restimulated with Mt Hsp65, but not Mt immunisation primed for responses to epitopes 86-100 and 396-410.

We generated αβ TCR⁺CD4⁺, RT1.B¹-restricted T cell lines specific for eight of the nine epitopes identified. Of these eight T cell lines two, H.36 and H.46 (which recognise 176-190 and 226-235 respectively) responded strongly to Mt while the others responded relatively weakly. This is consistent with the dominance of 176-190 following immunisation with Mt.

The relatively low quantities of hsp65 present in the Mt preparation might result in focusing of T cell responses on hsp65 epitopes with higher affinities for MHC class II molecules. Preliminary experiments testing the ability of peptides containing hsp65 epitopes to inhibit proliferation of T cell lines with other Ag specificities suggest that peptide 176-190 may have a higher affinity for RT1.B¹ molecules than do peptides 211-225 or 226-240 (data not shown). Thus, when Ag concentrations are limited (i.e. following Mt immunisation) dominance of T cell epitopes might be more dependent on their relative MHC binding affinities, whereas following immunisation with large quantities of specific antigen (50-100 µg hsp65 per rat) relative dominance might not be simply a function of affinity for MHC. Clearly, antigen processing of the intact hsp65 molecule will determine the peptide fragments generated during *in vivo* priming, and the naturally processed fragments will not be identical to the synthetic 15mers used in this study. Also the molecular context of hsp65 (i.e recombinant monomeric hsp65, or the multimeric native protein in the Mt preparation) could affect antigen processing and influence the pattern of epitope recognition.

Significantly, the dominant hsp65 epitope following Mt immunisation, 176-190, contains the 180-188 sequence previously reported to be recognised by the arthritogenic T cell clone A2b. Therefore, the AA-inducing protocol results in a T cell response skewed towards the AA-associated epitope. For PLNC responses, the longer 176-190 peptide induced stronger proliferative responses than the minimal 180-188 peptide. This is a significant finding as previous studies have analysed polyclonal responses to 180-188 (7,22). The length of naturally processed peptides found in the binding groove of MHC class II molecules has been described as 13 to 25 amino acids (23.24). Thus a more stable interaction of the 15mer 176-190 with the RT1.B¹ molecule compared with the 9mer 180-188 might account for the increased stimulatory activity.

Of the nine hsp65 T cell epitopes defined by this study, one was cross-reactive with rat hsp60. Although no significant response to any rat hsp60 peptide was observed at the PLNC level following immunisation with either hsp65 or Mt, the T cell line H.52, specific for the 256-265 epitope did respond to the corresponding rat hsp60 peptide. Accordingly this region is highly conserved (mycobacterial: ALSTLVVNKI, rat: ALSTLVLNRL) with seven residues identical and conservative substitutions at the other three positions. The region 243-265 shows highest identity between mycobacterial and mammalian hsp60s, with 18/23 residues identical and five conservative substitutions (11).

The present findings provide important insights into T cell mediated protective effect of hsp65 preimmunisation in experimental models of arthritis, for which three possible mechanisms have been proposed. Firstly, previous data suggested that hsp65 preimmunisation might down-regulate the response to the AA-associated 180-188 epitope (22). The results of the present study do not support this, as 176-190 is a co-dominant epitope following hsp65 immunisation.

Secondly, enhanced activation of T cells (recognising one or more hsp65 epitope) following preimmunisation might result in a more efficient recognition and clearance of Mt on subsequent challenge, before AA can develop. It was found that hsp65-immunisation results in improved recognition of hsp65 epitopes compared to Mt-immunisation (both in terms of number of epitopes recognised and magnitude of responses). These differences might form the basis for such a protective mechanism.

Reports of T cell recognition of epitopes conserved between mycobacterial hsp65 and mammalian hsp60 (14-17) have led to a third hypothesis, in which preimmunisation with hsp65 activates T cells recognising cross-reactive epitopes (16). Subsequent recognition of self hsp60 upregulated during inflammation within the joint would then regulate the inflammatory process, preventing development of chronic arthritis. If this hypothesis is correct, the relevant T cell epitope (for models using Lewis rats) must be residues 256-265 as this is the only cross-reactive epitope recognised following hsp65 immunisation. Interestingly, epitope 256-265 was recognised only poorly by Mt/IFA immunisation. Also, line H.52 responded weakly to Mt. Therefore the AA-inducing protocol is poor at activating T cells specific for this epitope.

Preimmunisation with hsp65 not only protects against AA, but also against arthritis induced without mycobacteria (6-10) and, in pristane (10) and CP20961-induced (6) arthritis, without any exogenously added protein. Therefore it is probable that the pathogenic mechanisms in these models differ, yet all can be prevented by preimmunisation with hsp65. With this in mind, the hypothesis which does not require recognition of whole Mt, but involves cross-reactive T cell recognition of mycobacterial hsp65 and rat hsp60 seems most attractive as protection could be accounted for by a single mechanism, regardless of the arthritogenic agent used. T cell recognition of self hsp60 might also be relevant in resistance to human arthritic conditions. T cell reactivity to self hsp60 has been reported in patients with RA (25) and JCA (26). An exciting extension of this was the report of two CD4⁺ T cell clones from a JCA patient which recognised cross-reactive epitopes in the highly conserved region (243-265) recognised by the H.52 T cell line in our study, and that the donor patient had a favourable outcome of disease (17). Also epitopes in this region of self hsp60 were recognised by CD4⁺ CTL from healthy human donors (14).

T cell reactivity against hsp65 is believed to be involved in the protective mechanism(s). Rats were preimmunised with synthetic peptides containing individual epitopes seven days prior to AA induction with Mt. This approach led to a striking protective effect in rats preimmunised with peptide 256-270. None of the other peptides tested showed any influence on AA development. The T cell line H.52, originally generated from hsp65 immunised rats and specific for epitope 256-265, also showed a protective effect on AA development when injected i.v. at Mt administration. Transfer of line H.52 did not induce total protection against AA but clearly reduced the severity of the arthritis. The low numbers of cells transferred in this study (5x10⁶ compared with 5x10⁷-10⁸ in other studies), enforced due to the slow rate of growth displayed by H.52, might be insufficient to induce a fully protective effect.

The finding that preimmunisation with peptide 176-190 (containing the AA-associated 180-188 epitope) had no effect on AA onset is in contrast with previous reports that preimmunisation with peptides 180-188 induces effective protection against AA. A possible explanation for this difference lies in the different preimmunisation regimens adopted. We immunised with peptide in the footpads seven days prior to Mt immunisation using DDA as adjuvant, whereas the previous studies immunised i.p. with peptide in IFA on days -35, -20 and -5. Although i.p. immunisation with peptide in IFA has been used to induce epitope specific T cell tolerance, this approach was reported to induce 180-188 specific T cells capable of transferring protection to naive rats. These findings could not be repeated.

No protective effect was found of administering the 180-188-specific T cell line H.36 at the time of Mt immunisation. This contrasts with the previously described protective effects of transferring spleen T cells from 180-188 immunised rats or the 180-188 specific T cell lines A2 and A2c. H.36 might therefore be an "A2b-like" line with AA inducing rather than protective activity, although transfer of H.36 did not increase severity of AA and we have not tested the ability of the line to induce AA in irradiated rats. Alternatively, the low numbers of cells transferred in this study again might not be sufficient to induce a protective effect.

Line H.52 has been shown to recognise the 256-270 sequence of rat hsp60. Similarly, short term T cell lines derived from rats immunised with peptide 256-270 (generated both from rats ten days after peptide immunisation and from protected rats 42 days after Mt administration) responded to the corresponding rat hsp60 peptide. These lines also showed small but significant responses to heat shocked APC indicating that the endogenous self hsp60 epitope could be presented in association with MHC class II for T cell recognition when hsp60 levels were upregulated.

Thus, immunisation with mycobacterial hsp65 256-270 protected against AA development and activated T cells capable of responding to the shared epitope in rat hsp60. This finding provides support for the hypothesis that the mechanism by which hsp65 preimmunisation protects Lewis rats against arthritis is based on activation of T cells that recognise an epitope shared with rat hsp60. Recognition by these T cells of elevated levels of the self epitope presented by MHC class II expressing cells at the site of inflammation (the joint) would then provide an antigen-specific mechanism for regulation of the inflammatory process. This mechanism does not require the "protective" T cell to recognise a mycobacterial component and therefore provides an attractive single mechanism for explaining hsp65-induced resistance to models not employing bacterial derived-arthritogens. The most notable of these models in the Lewis rat is the model induced with CP20961, which is a lipoidal amine and therefore has no possible antigenic cross-reactivity with hsp65. It was found indeed that peptide 256-270 confers protection against the CP20961-induced model.

Preimmunisation with rat 256-270 failed to protect against AA. T cell lines derived from rats immunised with M.256-270 and R.256-270 recognized two distinct epitopes. Immunisation with M.256-270 induced RT1.B¹-restricted T cells specific for the cross-reactive core epitope 256-265. Immunisation with R.256-270 induced RT1.D¹-restricted T cells specific for the rat-unique core epitope 261-270. Thus the protective effect observed here required T cell recognition of the 256-265 epitope in association with RT1.B¹. The relatively weak responses to R.256-270 after M.256-270 immunisation suggest the trimolecular interaction of R.256-270, RT1.B¹, and TCR was of relatively low affinity. The strong proliferative responses of the R.256-270 specific T cell line suggest that the R.256-270, RT1.D¹, TCR interaction might be of high affinity. The rat-unique R.261-270 epitope might be cryptic (i.e. not expressed with MHC class II after processing of endogenous hsp60 by APC and therefore not available for recognition by potentially protective T cells). If so, T cells with high affinity for this epitope would not be negatively selected in the thymus and would dominate the T cell response after immunisation with R.256-270, preventing development of an effective response to the protective 256-265 epitope. If differing affinities of the two interactions result from RT1.D¹ having higher binding affinity than RT1.B¹ for R.256-270, a form of "determinant capture" (Deng et al., J. Exp. Med. 178: 1675-1680) could account for this effect. Analysis of the binding affinities of both MHC class II molecules for R.256-270 and M.256-270 will clarify this. Thus therapeutic applications may require the use of bacterial hsp60 epitopes that induce relatively low affinity cross-reactive responses to endogenously processed self hsp60. Any self hsp60 epitopes that prove strongly immunogenic when administered as synthetic peptides may well be cryptic as responses to epitopes of naturally processed endogenous hsp60 would be controlled by normal mechanisms of thymic selection and peripheral tolerance. References
1. Pearson, C. M. 1956. Development of arthritis, periarthritis and periostitis in rats given adjuvant. *Proc*. *Soc. Exp. Biol. Med. 91:101.*
2. Pearson, C. M. and F. D. Wood. 1964. Passive transfer of adjuvant arthritis by lymph node or spleen cells. *J. Exp. Med. 120:547.*
3. Holoshitz, J., Y. Naparstek, A. Ben-Nun and I. R. Cohen. 1983. Lines of T lymphocytes induce or vaccinate against autoimmune arthritis. *Science. 219:56.*
4. Van Eden, W., J. Holoshitz, Z. Nevo, A. Frenkel, A. Klajman and I. R. Cohen. 1985. Arthritis induced by a T-lymphocyte clone that responds to *Mycobacterium tuberculosis* and to cartilage proteoglycans. *Proc. Natl. Acad. Sci*. *USA. 82:5117.*
5. Van Eden, W., J. E. R. Thole, R. van der Zee, A. Noordzij, J. D. A. van Embden, E. J. Hensen and I. R. Cohen. 1988. Cloning the mycobacterial epitope recognised by T lymphocytes in adjuvant arthritis. *Nature 331:171.*
6. Billingham, M. E. J., S. Carney, R. Butler and M. J. Colston. 1990. A mycobacterial heat shock protein induces antigen-specific suppression of adjuvant arthritis, but is not itself arthritogenic. *J. Exp. Med. 171:339.*
7. Hogervorst, E. J. M., J. P. A. Wagenaar, C. J. P. Boog, R. van der Zee, J. D. A. van Embden and W. van Eden. 1992. Adjuvant arthritis and immunity to the mycobacterial 65kD heat shock protein. *Intern. Immunol. 4:719*.
8. Van den Broek, M. F., E. J. M. Hogervorst. M. C. J. van Bruggen, W. van Eden, R. van der Zee and W. B. van der Berg. 1989. Protection against streptococcal cell wall-induced arthritis by pretreatment with the 65-kD mycobacterial heat shock protein. *J. Exp. Med. 170:449.*
9. Ito, J., C. J. Krco, D. Yu, H. S. Luthra and C. S. David. 1991. Preadministration of a 65KDa heat shock protein, GroEL, inhibits collagen induced arthritis in mice. *J*. *Cell. Biochem. 15A:284.*
10. Thompson, S. J., G. A. W. Rook, R. J. Brealey, R. van der Zee and C. J. Elson. 1990. Autoimmune reactions to heat-shock proteins in pristane-induced arthritis. *Eur. J. Immunol*. *20:2479*.
11. Jindal, S., A. K. Dubani, B. Singh C. B. Harley and R. S. Gupta. 1989. Primary structure of a human mitochondrial protein homologous to the bacterial and plant chaeronins and to the 65-kilodalton mycobacterial antigen. *Mol. Cell. Biol. 9:2279.*
12. Karlsson-Parra, A., K Soderstrom, M. Ferm, J. Ivanyi, R. Kiessling and L. Klareskog. 1990. Presence of human heat shock protein (hsp) in inflamed joints and subcutaneous nodules of RA patients. *Scand. J. Immunol. 31:283.*
13. Boog, C. J. P., E. R. de Graeff-Meeder, M. A. Lucassen, R. van der Zee, M. M. Voorhorst-0gink, P. J. S. van Kooten, H. J. Geuze and W. van Eden. 1992. Two monoclonal antibodies generated against human hsp60 show reactivity with synovial membranes of patients with juvenile chronic arthritis. *J. Exp. Med. 175:1805.*
14. Munk, M. E., B. Schoel, S. Modrow, R. W. Karr, R. A. Young and S. H. E. Kaufmann. 1989. T lymphocytes from healthy individuals with specificity to self-epitopes shared by the mycobacterial and human 65-kilodalton heat shock protein. *J. Immunol. 143:2844.*
15. Lamb, J. R., V. Bal, P. Mendez-Samperio, A. Mehlert, A. So, J. Rothbard, S. Jindal, R. A. Young and D. B. Young. 1989. Stress proteins may provide a link between the immune response to infection and autoimmunity. *Intern. Immunol. 1:191.*
16. Anderton, S. M. , R. van der Zee and J. A. Goodacre. 1993. Inflammation activates self hsp60-specific T cells. *Eur. J. Immunol. 23:33.*
17. Quayle, A. J., K. B. Wilson, S. G. Li, J. Kjeldsen-Kragh, F. Of tung , T. Shinnick, M. Sioud, 0. Forre, J. D. Capra and J. B. Natvig. 1992. Peptide recognition, T cell receptor usage and HLA restriction elements of human heat-shock protein (hsp) 60 and mycobacterial 65-kDa hsp-reactive T cell clones from rheumatoid synovial fluid. *Eur. J. Immunol. 22:1315.*
18. Snippe, H., and C. H. Kaaieveld. 1989. The immunoadjuvant dimethyl dioctadecyl ammonium bromide. In: *Immunological adjuvants and vaccines, vol. 179*. G. Gregoriades, A. C. Allison. P. Post, eds. Plenum Press, New York. p.47.
19. Van der Zee, R., M. H. M. Wauben, T. H. A. Lots and W. van Eden. 1992. Simultaneous multiple peptide synthesis (SMPS) for the analysis of T cell epitopes. *J. Cell. Biochem. 16D:83.*
20. Shinnick, T. M. 1987. The 65-kilodalton antigen of *Mycobacterium tuberculosis. J. Bactertiol. 169:1080.*
21. Venner, T. J. and R. S. Gupta. 1990. Nucleotide sequence of rat hsp60 (chaperonin, GroEL homolog) cDNA. *Nucleic Acids Res. 18:5309.*
22. Hogervorst, E. J. M., C. J. P. Boog, J. P. A. Wagenaar, M. H. M. Wauben, R. van der Zee and W. van Eden. 1991. T cell reactivity to an epitope of the mycobacterial 65-kDa heat shock protein (hsp65) corresponds with arthritis susceptibility in rats and is regulated by hsp65-specific cellular responses. *Eur. J. Immunol. 21:1289.*
23. Rudensky, A. Y., P. Preston-Hurlburt. S. C. Hong, A. Barlow and C. A.Janeway. 1991. Sequence analysis of peptides bound to MHC class II molecules. *Nature. 353:622.*
24. Chicz, R. M., R. G. Urban, W. S. Lane, J. C. Gorga L. J. Stern, D. A. A. Vignali and J. L. Strominger. 1993. Predominant naturally processed peptides bound to HLA-DR1 are derived from MHC-related molecules and are heterogeneous in size. *Nature. 358:764.*
25. Pope, R. M., R. M. Lovis and R. S. Gupta. 1992. Activation of synovial fluid T lymphocytes by 60-kD heat-shock proteins in patients with inflammatory synovitis. *Arthritis Rheum. 35:43.*
26. De Graeff-Meeder, E. R., R. van der Zee, G. T. Rijkers, H-J. Schuurman, W. Kuis, J. W. J. Bijlsma, B. J. M. Zegers and W. van Eden. 1991. Recognition of human 60 kD heat shock protein by mononuclear cells from patients with juvenile chronic arthritis. *Lancet. 337:1368.*

### Description of the figures

**Figure 1:** Mt or hsp65 immunisation primes for T cell recognition of hsp65.
   Ten day PLNC from rats immunised with hsp65, Mt/IFA, or Mt/DDA were tested against hsp65, PPD and Mt over a range of concentrations (20 µg/ml, which gave maximal responses, shown here). Unimmunised splenocytes and PLNC from rats immunised with PBS/DDA were used as negative controls (PLNC from IFA immunised rats showed the same response pattern as PBS/DDA-PLNC). All S.E.M's were less than 20%.
**Figure 2:** Anti-MHC mAb inhibition of anti-hsp65 PLNC responses.
   Ten day hsp65-PLNC were cultured with a range of hsp65 doses in the presence of 10 µg/ml mAb specific for RT1.B (0X6), RT1.D (0X17) and RT1.A (0X18). Anti-cmp = UD15 anti-chloramphenicol, isotype control antibody. All S.E.M's were less than 20%.
**Figure 3:** Responses to mycobacterial hsp65 peptides following immunisation with hsp65.
   Responses to the entire panel of hsp65 peptides were analysed using ten day hsp65-PLNC (Fig. 3a), or an hsp65-specific T cell line (Fig. 3b), generated from the same hsp65-PLNC population. Peptides were tested at 5 and 20 µg/ml (20 µg/ml shown here). Responses to hsp65, PPD and Mt (20µg/ml) were all greater than 60,000 cpm in (a) and 150,000 cpm in (b). Peptides inducing significant responses are identified. Where overlapping peptides were stimulatory, the peptide giving stronger responses is identified. The results of this experiment were reproduced in three further experiments. All S.E.M's were less than 20%.

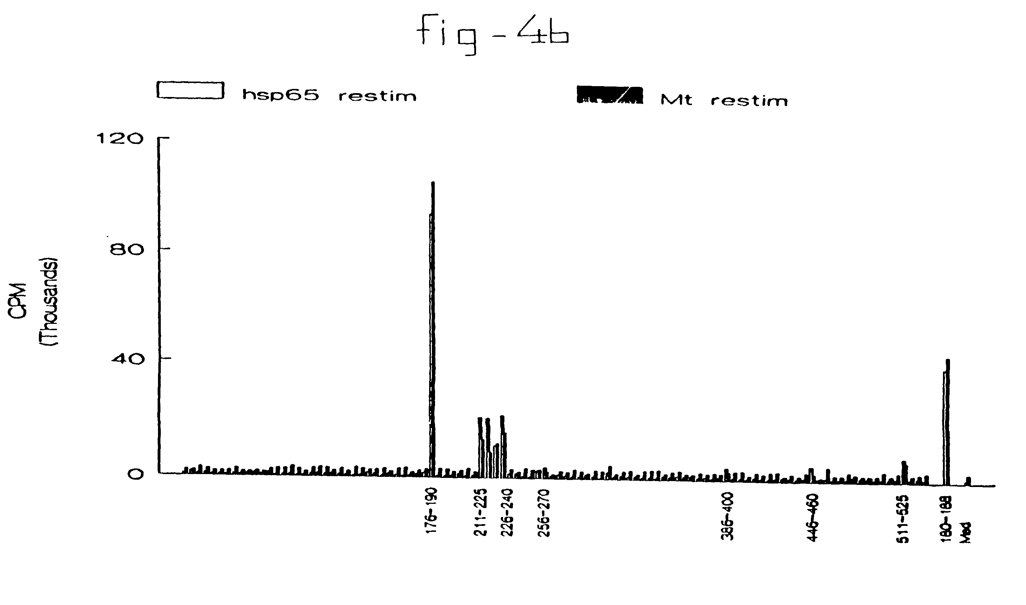

**Figure 4:** Responses to mycobacterial hsp65 peptides following immunisation with whole Mt.
   Responses to the entire panel of hsp65 peptides were analysed using ten day PLNC from rats immunised with Mt/DDA (Fig. 4a), or T cell lines generated from Mt/IFA-PLNC by restimulation with hsp65 or Mt (Fig. 4b). Peptides were tested at 5 and 20 µg/ml (20 µg/ml gave stronger responses, and is shown here). Responses to hsp65, PPD and Mt (20µg/ml) were all greater than 40,000 cpm in (a) and 150,000 cpm in (b). Peptides inducing significant responses are highlighted. All S.E.M's were less than 20%.
**Figure 5:** Response of T cell lines specific for defined hsp65 epitopes.
   T cell lines were tested for responses to overlapping hsp65 peptides, corresponding rat hsp60 peptides, hsp65 and Mt over a range of Ag concentrations (10 µg/ml shown here) (Fig's 5.1 to 5.4). All lines responded to PPD and none responded to control hsp65 peptides containing other epitopes. All S.E.M's were less than 20%.
**Figure 6:** Anti-MHC mAb inhibition of T cell line responses.
   Epitope-specific and hsp65-specific T cell lines were cultured with irradiated APC and 1 µg/ml Ag (specific peptide for epitope-specific lines or hsp65 for the hsp65-specific line) in the presence of 10 µg/ml mAb specific for RT1.B (0X6), RT1.D (0X17) and RT1.A (0X18). Anti-cmp = UD15 anti-chloramphenicol, isotype control antibody. All S.E.M's were less than 20%.
**Figure 7:** Modulation of AA development by preimmunisation with hsp65 peptides.
   Rats were immunised in the hind footpads with 100 µg of individual synthetic peptides or PBS in DDA seven days prior to AA induction using 5mg Mt in 100 µl IFA injected i.d. at the base of the tail (Fig's 7.1 to 7.4). Five rats were used in each preimmunisation group. Arthritis scores were assessed daily from eight days after Mt injection.
**Figure 8:** PLNC responses of peptide-preimmunised AA rats.
   Rats were preimmunised and Mt-immunised as described in Fig. 7. PLNC (pooled inguinal and popliteal LN) were isolated 42 days after Mt-immunisation and tested for responses to hsp65 peptides containing defined T cell epitopes (20µg/ml). All PLNC responded to hsp65, Mt (responses were all greater than 50,000 cpm). All SEM were less than 20%.
**Figure 9:** Modulation of AA using epitope-specific T cell lines.
   Rats were administered with 5x10⁶ T cells i.v. in PBS or PBS alone at the time of AA induction using 5 mg Mt in 100 µl IFA injected i.d. at the base of the tail. Five rats were used in each group. Arthritis scores were assessed daily from eight days after Mt injection. Results using lines H.46 (specific for 226-235) and H.52 (specific for 256-265) are shown. Injection of lines H.36 and H.43 (specific for epitopes 180-188 and 216-225 respectively) had no significant effect on AA (data not shown).
**Figure 10**: Modulation of CP20961-induced arthritis by preimmunisation with hsp65 peptides.
   Rats were immunised in the hind footpads with 100 µg of individual synthetic peptides (211-225 or 256-270) or PBS in DDA seven days prior to AA induction using 2 mg CP20961 in 100 µl mineral oil injected i.d. at the base of the tail. Five rats were used in each preimmunisation group. Arthritis scores were assessed daily from eight days after Mt injection.
**Figure 11:** Preimmunisation with rat hsp60 (256-270) does not protect against AA.
   Rats were immunised in the hind footpads with 100 µg of individual synthetic peptides or PBS in DDA seven days prior to AA induction using 5 mg Mt in 100 µl IFA injected i.d. at the base of the tail. Five rats were used in each preimmunisation group. Arthritis scores were assessed daily from eight days after Mt injection. Preimmunisation with a control peptide (hsp65 211-225) did not influence AA development (data not shown).
Figure 12: Immunisation with hsp65(256-270) and rat hsp60(256-270) primes for T cell responses to distinct epitopes.
   Rats were immunised in the hind footpads with either M.256-270 or R.256-270 in DDA. Seven days later PLNC were isolated and restimulated *in vitro* with the immunised peptide. The resulting T cell lines (line P.m52 recognising M.256-270, and line P.r57 recognising R.256-270) were tested for responses to mycobacterial and rat peptides in the presence of irradiated syngeneic spleen APC. All SEM were less than 20%.
Figure 13: Multisequence alignment of human, rat, mouse and M. Bovis BCG heat shock protein hsp65 (hsp60) in one-letter code.
   The alignment was done on 4 protein sequences. An asterix ^{*} shows perfectly conserved aminoacids. A dot . shows well-conserved, i.e. similar although not identical aminoacids.

- Consensus length:: 573
- Identity :: 254 ( 44.3%)
- Similarity:: 211 ( 36.8%)

### Dictionary of the sequences used for the alignment

[1] P60$HUMAN Size: 573 residues.
   - DE: Mitochondrial matrix protein P1 precursor (P60 lymphocyte protein) (Chaperonin homologue) (HUCHA60) (Heat shock protein 60) (hsp60).
   - OS: HUMAN (Homo sapiens).
[2] P60$RAT Size: 547 residues.
   - DE: Mitochondrial matrix protein P1 (P60 lymphocyte protein) (Chaperonin homologue) (heat shock protein 60) (hsp60).
   - OS: RAT (Rattus norvegicus).
[3] P60$MOUSE Size: 555 residues.
   - DE: Mitochondrial matrix protein P1 precursor (P60 lymphocyte protein) (Chaperonin homologue) (heat shock protein 60) (hsp60) (fragment).
   - OS: MOUSE (Mus musculus).
[4] P60$M.TUB Size: 540 residues. Mycobacterium tuberculosis / Mycobacterium bovis BCG hsp60

## Claims

1. Use of a peptide comprising a series of up to 30 aminoacids of the aminoacid sequence of a mycobacterial heat-shock protein selected from hsp65, hsp60 (GroEL), DnaJ, hsp70 family (DnaK), ubiquitin, hsp10 (GroES), hsp20-30, hsp47, hsp56, TCP-1 (T complex peptide), hsp90 and hsp104/110,
said mycobacterial heat-shock protein having a conserved mammalian stress protein homologue, wherein the overall aminoacid sequence identity between the mycobacterial and the mammalian homologues is at least 25%, the sequence identity between the mycobacterial and the mammalian homologues of an area of at least 75 consecutive aminoacids is at least 40%,
said peptide containing a T cell epitope of said heat-shock protein and comprising at least 5 aminoacids which are identical with the corresponding aminoacids in the same relative position in said T cell epitope, at least 4 consecutive aminoacids of said at least 5 aminoacids being identical with the corresponding mammalian stress protein aminoacids, for preparing a pharmaceutical composition for the treatment or the prevention of inflammatory diseases, including autoimmune diseases.

2. Use according to claim 1, wherein the overall aminoacid sequence identity between the microbial and the mammalian homologues is at least 40% and the sequence identity between the microbial and the mammalian homologues of an area of at least 75 consecutive aminoacids is at least 50%.

3. Use according to claim 2, wherein said heat-shock protein is heat shock protein hsp65 of *Mycobacterium tuberculosis* (identical to hsp65 of *M. bovis* BCG) as depicted in SEQ ID No. 1.

4. Use according to claim 3, wherein the peptide comprises at least 5 aminoacids which are identical with the corresponding aminoacids in the same relative position in one of the sequences 81-100 and 241-270 of SEQ ID No. 1.

5. Use according to claim 4, wherein the peptide comprises at least 5 aminoacids which are identical with the corresponding aminoacids in the same relative position in one of the sequences 84-95 and 256-265 of SEQ ID No. 1.

6. Use according to any one of claims 1-5, wherein one or more of the aminoacid residues has been exchanged with a residue of an aminoacid having similar size, charge and polarity, or with aminoacid mimetics resulting in one or more backbone modifications.

7. Use of a nucleotide sequence encoding a peptide as defined in any one of claims 1-5, or a vector containing said nucleotide sequence, for preparing a pharmaceutical composition for the treatment or the prevention of inflammatory diseases, including autoimmune diseases.

8. Use according to any one of claims 1-7, for preparing a pharmaceutical composition for the treatment or the prevention of diabetes, arthritic diseases, atherosclerosis, multiple sclerosis and/or myasthenia gravis.

9. Method of producing a peptide suitable in the prevention or treatment of inflammatory diseases, comprising the steps of:
a) selecting a mycobacterial heat-shock protein having a conserved mammalian stress protein homologue, wherein the overall aminoacid sequence identity between the mycobacterial and the mammalian homologues is at least 25%, and the sequence identity between the mycobacterial and the mammalian homologues of an area of at least 75 consecutive aminoacids is at least 40%;
b) preparing peptides of up to 30 aminoacids of said mycobacterial heat-shock protein, at least 5 of which are identical with the corresponding aminoacids in the same relative position in said stress protein, of which a series of at least 4 consecutive aminoacids is identical both to a series of aminoacids of the selected mycobacterial protein and to the corresponding series of mammalian stress protein aminoacids;
c) screening the prepared peptides for the presence of a T cell epitope and selecting the peptides on which a T cell epitope is present.

10. Use of a peptide as defined in any one of claims 1-6 or of an antibody raised against said peptide for preparing a diagnostic composition for the diagnosis of inflammatory diseases, including autoimmune diseases.

## Patentansprüche

1. Verwendung eines Peptids, umfassend eine Folge von bis zu 30 Aminosäuren der Amiriosäuresequenz eines mycobakteriellen Hitzeschockproteins, ausgewählt aus hsp65, hsp60 (GroEL), DnaJ, der hsp70-Familie (DnaK), Ubiquitin, hsp10 (GroES), hsp20-30, hsp47, hsp56, TCP-1 (T-Komplexpeptid), hsp90 und hsp104/110,
wobei dieses mycobakterielle Hitzeschockprotein ein konserviertes Säugerstressproteinhomolog hat, worin die gesamte Aminosäuresequenzidentität zwischen den mycobakteriellen und den Säugerhomologen wenigstens 25 % beträgt, wobei die Sequenzidentität zwischen den mycobakteriellen und den Säugerhomologen eines Bereichs von wenigstens 75 aufeinanderfolgenden Aminosäuren wenigstens 40 % beträgt, wobei dieses Peptid ein T-Zellepitop dieses Hitzschockproteins enthält und wenigstens 5 Aminosäuren umfasst, die identisch sind mit den korrespondierenden Aminosäuren, die sich in derselben relativen Position in diesem T-Zellepitop befinden, wobei wenigstens 4 aufeinanderfolgende Aminosäuren dieser wenigstens 5 Aminosäuren identisch sind mit den korrespondierenden Säugerstressproteinaminosäuren,
zur Herstellung einer pharmazeutischen Zusammensetzung für die Behandlung oder die Vorbeugung von Entzündungserkrankungen, einschließlich Autoimmunerkrankungen.

2. Verwendung nach Anspruch 1, worin die gesamte Aminosäuresequenzidentität zwischen den mikrobiellen und den Säugerhomologen wenigstens 40 % beträgt und die Sequenzidentität zwischen den mikrobiellen und den Säugerhomologen eines Bereichs von wenigstens 75 aufeinanderfolgenden Aminosäuren wenigstens 50 % beträgt.

3. Verwendung nach Anspruch 2, worin dieses Hitzeschockprotein das Hitzeschockprotein hsp65 von *Mycobacterium tuberculosis* (identisch mit hsp65 von *M. bovis* BCG) ist, wie veranschaulicht in SEQ ID No. 1.

4. Verwendung nach Anspruch 3, worin das Peptid wenigstens 5 Aminosäuren umfasst, die identisch sind mit den korrespondierenden Aminosäuren in derselben relativen Position in einer der Sequenzen 81-100 und 241-270 von SEQ ID No. 1.

5. Verwendung nach Anspruch 4, worin das Peptid wenigstens 5 Aminosäuren umfasst, die identisch sind mit den korrespondierenden Aminosäuren in derselben relativen Position in einer der Sequenzen 84-95 und 256-265 von SEQ ID No. 1.

6. Verwendung nach einem der Ansprüche 1-5, worin einer oder mehrere der Aminosäurereste mit einem Rest einer Aminosäure mit ähnlicher Größe, Ladung und Polarität, oder mit Aminosäuremimetika ausgetauscht wurde, was in einer oder mehreren Modifikationen des Grundgerüstes resultiert.

7. Verwendung einer Nukleotidsequenz, die ein Peptid kodiert, wie es in einem der Ansprüche 1-5 definiert ist, oder Verwendung eines Vektors, der diese Nukleotidsequenz enthält, zur Herstellung einer pharmazeutischen Zusammensetzung für die Behandlung oder die Vorbeugung von Entzündungserkrankungen, einschließlich Autoimmunerkrankungen.

8. Verwendung nach einem der Ansprüche 1-7 zur Herstellung einer pharmazeutischen Zusammensetzung für die Behandlung oder die Vorbeugung von Diabetes, arthritischen Erkrankungen, Atherosklerose, Multiple Sklerose und/oder Myasthenia gravis.

9. Verfahren zur Herstellung eines Peptids, das geeignet ist für die Vorbeugung oder Behandlung von Entzündungserkrankungen, umfassend die folgenden Schritte:
a) Auswählen eines mycobakteriellen Hitzeschockproteins mit einem konservierten Säugerstressproteinhomolog, worin die gesamte Aminosäuresequenzidentität zwischen den mycobakteriellen und den Säugerhomologen wenigstens 25 % beträgt, und wobei die Sequenzidentität zwischen den mycobakteriellen und den Säugerhomologen eines Bereichs von wenigstens 75 aufeinanderfolgenden Aminosäuren wenigstens 40 % beträgt;
b) Herstellen von Peptiden von bis zu 30 Aminosäuren dieses mycobakteriellen Hitzeschockproteins, wobei wenigstens 5 davon identisch sind mit den korrespondierenden Aminosäuren in derselben relativen Position in diesem Stressprotein, von denen eine Folge von wenigstens 4 aufeinanderfolgenden Aminosäuren identisch ist sowohl mit einer Folge von Aminosäuren des ausgewählten mycobakteriellen Proteins als auch mit der korrespondierenden Folge von Säugerstressproteinaminosäuren;
c) Screenen der hergestellten Peptide für die Anwesenheit eines T-Zellepitops und Auswählen der Peptide, bei denen ein T-Zellepitop vorhanden ist.

10. Verwendung eines Peptids, wie in einem der Ansprüche 1-6 definiert, oder eines Antikörpers, der gegen dieses Peptid erzeugt wurde, zur Herstellung einer diagnostischen Zusammensetzung für die Diagnose von Entzündungserkrankungen, einschließlich Autoimmunerkrankungen.

## Revendications

1. Utilisation d'un peptide comprenant une série de jusqu'à 30 acides aminés de la séquence d'acides aminés d'une protéine de choc thermique mycobactérienne sélectionnée parmi hsp65, hsp60 (GroEL), DnaJ, la famille hsp70 (DnaK), ubiquitine, hsp10 (GroES), hsp20-30, hsp47, hsp56, TCP-1 (peptide complexe T), hsp90 et hsp104/110,
ladite protéine de choc thermique mycobactérienme ayant un homologue de protéine de stress de mammifères conservé, dans laquelle l'identité de séquence d'acides aminés d'ensemble entre les homologues mycobactérien et de mammifères est d'au moins 25%, l'identité de séquence entre les homologues mycobactérien et de mammifères d'une zone d'au moins 75 acides aminés consécutifs est d'au moins 40%,
ledit peptide contenant un épitope de cellule T de ladite protéine de choc thermique et comprenant au moins 5 acides aminés qui sont identiques aux acides aminés correspondants dans la même position relative dans ledit épitope de cellule T, au moins 4 acides aminés consécutifs desdits au moins 5 acides aminés étant identiques aux acides aminés de protéine de stress de mammifères correspondants, pour préparer une composition pharmaceutique pour le traitement ou la prévention de maladies inflammatoires, y compris les maladies auto-immunes.

2. Utilisation selon la revendication 1, dans laquelle l'identité de séquence d'acides aminés d'ensemble entre les homologues microbien et de mammifères est d'au moins 40% et l'identité de séquence entre les homologues microbien et de mammifères d'une zone d'au moins 75 acides aminés consécutifs est d'au moins 50%.

3. Utilisation selon la revendication 2, dans laquelle ladite protéine de choc thermique est la protéine de choc thermique hsp65 de la tuberculose mycobactérienne (identique à hsp65 de *M. bovis* BCG) comme représentée dans le numéro d'ID de SEQ 1.

4. Utilisation selon la revendication 3, dans laquelle le peptide comporte au moins 5 acides aminés qui sont identiques aux acides aminés correspondants dans la même position relative dans l'une des séquences 81-100 et 241-270 du numéro d'ID de SEQ 1.

5. Utilisation selon la revendication 4, dans laquelle le peptide comprend au moins 5 acides aminés qui sont identiques aux acides aminés correspondants dans la même position relative dans l'une des séquences 84-95 et 256-265 du numéro d'ID de SEQ 1.

6. Utilisation selon l'une quelconque des revendications 1 à 5, dans laquelle l'un ou plusieurs des résidus d'acide aminé ont été remplacés par un résidu d'un acide aminé ayant une dimension, une charge et une polarité similaires, ou par des imitations d'acide aminé ayant pour résultat une ou plusieurs modifications de chaîne principale.

7. Utilisation d'une séquence de nucléotides codant un peptide selon l'une quelconque de revendications 1 à 5, ou un vecteur contenant ladite séquence de nucléotides, pour préparer une composition pharmaceutique pour le traitement ou la prévention de maladies inflammatoires, y compris les maladies auto-immunes.

8. Utilisation selon l'une quelconque de revendications 1 à 7, pour préparer une composition pharmaceutique pour le traitement ou la prévention du diabète, des maladies arthritiques, de l'athérosclérose, de la sclérose en plaques et/ou de la myasthénie grave.

9. Procédé pour produire un peptide convenant pour la prévention ou le traitement de maladies inflammatoires, comportant les étapes consistant à :
sélectionner une protéine de choc thermique mycobactérienne ayant un homologue de protéine de stress de mammifères conservé, dans lequel l'identité de séquence d'acides aminés d'ensemble entre les homologues mycobactérien et de mammifères est d'au moins 25%, et l'identité de séquence entre les homologues mycobactérien et de mammifères d'une zone d'au moins 75 acides aminés consécutifs est d'au moins 40%,
b) préparer des peptides de jusqu'à 30 acides aminés de ladite protéine de choc thermique dont au moins 5 acides aminés sont identiques aux acides aminés correspondants dans la même position relative dans ladite protéine de stress, dont une série d'au moins 4 acides aminés consécutifs est identique tant à une série d'acides aminés de la protéine mycobactérienne sélectionnée qu'à la série correspondante d'acides aminés de la protéine de stress de mammifères ;
c) examiner les peptides préparés quant à la présence d'un épitope de cellule T et sélectionner les peptides sur lesquels est présent un épitope de cellule T.

10. Utilisation d'un peptide selon l'une quelconque de revendications 1 à 6 ou d'un anticorps produit par croissance contre ledit peptide pour préparer une composition diagnostique pour le diagnostic de maladies inflammatoires, y compris les maladies auto-immunes.
